# EUROPEAN PATENT SPECIFICATION

(11) **EP 2 726 873 B1**
(45) Date of publication and mention of the grant of the patent: **09.08.2017**
(21) Application number: 12742947.0
(22) Date of filing: 26.06.2012
(51) Int. Cl.: G01N 33/543

(54) **MOLECULAR ARCHITECTURE ON MAGNETIC PARTICLES FOR AFFINITY ASSAYS WITH LOW NON-SPECIFIC BINDING**
MOLEKULARE ARCHITEKTUR VON MAGNETISCHEN PARTIKELN FÜR AFFINITÄTSPRÜFUNGEN MIT GERINGER NICHT SPEZIFISCHER BINDUNG
ARCHITECTURE MOLÉCULAIRE DE PARTICULES MAGNÉTIQUES POUR DES ANALYSES DÝAFFINITÉ AVEC UNE FAIBLE LIAISON NON SPÉCIFIQUE

(30) Priority: 30.06.2011 EP 11172054
(43) Date of publication of application: 07.05.2014
(73) Proprietor: Koninklijke Philips N.V., 5656 AE Eindhoven (NL)
(72) Inventor: RANZONI, Andrea, 5656 AE Eindhoven (NL); SABATTE, Gwénola, 5656 AE Eindhoven (NL); PRINS, Menno, Willem, Jose, 5656 AE Eindhoven (NL)
(74) Representative: Steffen, Thomas
(86) International application number: PCT/IB2012/053215
(87) International publication number: WO 2013/001447

(56) References cited:
- EP-A2- 0 617 284
- WO-A1-92/03730
- US-A- 4 894 253
- US-A- 5 212 063
- US-A1- 2002 128 234
- TOWNSEND ET AL: "Tetanus toxin C fragment-conjugated nanoparticles for targeted drug delivery to neurons", BIOMATERIALS, ELSEVIER SCIENCE PUBLISHERS BV., BARKING, GB, vol. 28, no. 34, 1 October 2007 (2007-10-01), pages 5176-5184, XP022282482, ISSN: 0142-9612, DOI: 10.1016/J.BIOMATERIALS.2007.08.011
- RANZONI A ET AL: "Frequency-selective rotation of two-particle nanoactuators for rapid and sensitive detection of biomolecules", NANO LETTERS 20110511 AMERICAN CHEMICAL SOCIETY USA, vol. 11, no. 5, 11 May 2011 (2011-05-11), pages 2017-2022, XP002663465, DOI: DOI:10.1021/NL200384P cited in the application
- GROLL JUERGEN ET AL: "Ultrathin coatings from isocyanate terminated star PEG prepolymers: patterning of proteins on the layers", LANGMUIR: THE ACS JOURNAL OF SURFACES AND COLLOIDS, AMERICAN CHEMICAL SOCIETY, WASHINGTON, DC, USA, vol. 21, no. 7, 29 March 2005 (2005-03-29) , pages 3076-3083, XP009107912, ISSN: 0743-7463, DOI: 10.1021/LA047438N

## Description

### FIELD OF THE INVENTION

The present invention relates to a coating for preventing non-specific binding of molecules to a surface layer comprising an affinity molecule, said coating comprises a shell structure, wherein said shell structure comprises a first layer comprising one or more affinity molecules and further a second layer, which is coupled to the first layer, and wherein said second layer comprises non-affine spacer molecules forming a mesh. The present invention further relates to a particle, a flat structure or a matrix comprising such a coating. In a further aspect the present invention relates to the use of the coating, coated particle, coated flat structure or coated matrix for detection and/or purification of a specific target biomolecule from a sample and/or determination of the concentration of the specific target biomolecule. The present invention further relates to an assay for the detection of specific target biomolecules from a sample and/or determination of the concentration of specific target biomolecules comprising the use of such coating, coated particle, coated flat structure or coated matrix, in particular coated nanoparticles.

### BACKGROUND OF THE INVENTION

The demand for pervasive and effective healthcare moves the world of *in vitro* diagnostics towards integrated random-access and point-of care solutions. The achievement of such solutions is demanding: the tests needs to be rapid, sensitive, quantitative and accurate. Moreover the platform on which the test is performed need to be easy to use and compact.

Affinity assays make use of biological molecules to capture specific target molecules from a sample and allow a determination of their concentration. Typically, affinity capture is achieved by dispersing nano- or microparticles coated with capture molecules into sample fluid (Luchini, A. et al., 2008, Nano Lett., 8(1), 350 -361). Typical affinity-based assays are therefore used in a huge number of applications such as diagnostic assays, detection of biomolecules in research such as proteins, peptides and nucleic acids thereby making use of affinity molecules such as, e.g. antibodies, which are typically characterized by a high binding affinity towards a specific biomolecule.

An important drawback of affinity-assays, however, is the fact that sensitivity is largely limited by non-specific binding. The main difficulty is to conceive assay technologies that are very sensitive and specific and that does not intrinsically suffer from high background signal from the sample fluid that is probed. Non-specific binding typically leads to an increased background signal and to inaccurate detection. In particular, non-specific binding is even more challenging when complex biological matrices such as human plasma are used as sample fluid.

In recent years, more accurate and sensitive solution-based affinity assays have been developed, which are based on the use of superparamagnetic nanoparticles for a novel generation of assays. For example, magnetic nanoparticles which are functionalized with affinity molecules capture target molecules and the formation of target-induced clusters and chains of particles is detected optically (WO 03/044532; Ranzoni et al., 2011, Nano Lett, 11, 2017-2022). However, especially in particle-based assays, important contributions to non-specific signals come from particle-particle interactions and particle-surface interactions. US 5 212 063 A1 discloses a process for the detection of analytes in body fluids containing free biotin by immunoassay with the use of biotin conjugates. The document mentions polymer particles consisting of a core and containing a polymer which has a plurality of binding sites for biotin and, as a covering, at least one layer of protein. Townsend et al., 2007, Biomaterials, 28(34), 5176-5184 discloses tetanus toxin C fragment-conjugated nanoparticles for target drug delivery to neurons. Groll et al., 2005, Langmuir, 21(7), 3076-3083 discloses ultrathin coatings from isocyanate terminated star PEG prepolymers. In particular, the document describes star PEG coatings, which form cross-linked network pattern on substrates. EP 0 617 284 A2 discloses an apparatus and process for qualitative and quantitative measurement of substances. The apparatus comprises a liquid permeable porous reaction membrane whose surface has one reaction area to which an affinity substance is immobilized, a porous body arranged on the upper part of the porous reaction membrane, to which a soluble agent is adhered, an absorption membrane and a liquid non-permeable transparent cover and case. US 2002/128234 discloses multifunctional, polyionic copolymers which are synthesized on substrate surfaces. The coatings are indicated as being useful for suppression of non-specific interactions, adsorption or attachment of molecular or ionic components present in an analyte solution. Further, WO 92/03730 discloses a biosensor for the detection of an analyte in solution including using enzyme-substrate interaction for response amplification, includes a coating layer comprising an immobilised first specific binding partner for the analyte, an enzyme-labelled reagent being either a second specific US4894253 discloses a coating comprising a shell structure with a polymer network functioning as size exclusion means. There is thus a need to design novel structures, which are capable of efficiently preventing or minimizing non-specific binding to a surface, especially a particle surface, coated with affinity-molecules while at the same time enhancing the specific affinity. There is also a strong need to avoid non-specific clustering of particles, which is a limiting factor in a huge number of detection assays.

### OBJECTS AND SUMMARY OF THE INVENTION

The present invention addresses these needs and provides means for reducing non-specific binding to particles, surfaces, and matrices. The above objective is in particular accomplished by a coating for preventing non-specific binding of molecules to a surface layer according to claim 1.
The ratio behind the present invention is to provide a molecular architecture of spacer molecules, which acts as barrier and thus generates a steric hindrance for non-specific molecules. On the other hand, if such a coating is provided on a particle such as a micro- or nanoparticle, the double-layer of non-affine spacer molecules functions as steric barrier and further generates entropic effects, preventing the particle surfaces to get in touch with each other. One major advantage is that the coatings of the present invention allow to effectively cover surfaces with a dense three-dimensional mesh of spacer molecules. Such coatings thus combine the advantageous properties of strong specific affinity and low non-specific binding.

In particular, it could be observed that the provision of a coating comprising a mesh of spacer molecules that is thicker than the surface roughness prevents non-specific binding and further enables effective capture of target molecules.

Without wishing to be bound to a theory, one explanation for the observed high specific affinity is that the incorporated capture molecules are present in a high density on the surface. Further, the affinity molecules which are embedded within a thick three-dimensional shell of spacer molecules can be effectively oriented away from the surface. Another reason could be that the affinity molecules are sufficiently mobile within the coating and sufficiently reach out in order to form biochemical interactions with the target molecules. Hence, the network of non-affine spacer molecules as described herein has the effect to balance and orientate the affinity molecules of the first layer from the surface away. The branches of spacer molecules thus form a dense mesh, which could act as a steric barrier between the affinity molecule and the surface.

In dose response assays it could, in particular, be shown that conventional particle surfaces, where the affinity-molecules such as antibodies are directly coupled without a linker and an additional shell structure, a considerable amount of surfactants is required in order to reduce the background level. In contrast, the coating architecture according to the present invention is capable of reducing the background level by a factor of 2 without the use of a surfactant. One major advantage over the art is further that the modified surface architecture improves the blank level in optomagnetic detection assays and thus improves the limit of detection.

In a preferred embodiment of the present invention the affinity molecule is selected from the group consisting of aptamers, peptides, proteins, oligonucleotides and molecular imprinted polymers.

In a further preferred embodiment of the present invention affinity molecule is an antibody or a fragment thereof.

In another preferred embodiment of the present invention the surface layer comprising an affinity molecule or the first layer is coupled to a particle surface, a flat surface, matrix, or a nanoparticle surface.

In a further preferred embodiment of the present invention the material of said surfaces or said matrix comprises a material selected from the group consisting of agarose, polystyrene, latex, polyvinyl alcohol, silica and a magnetic material.

In another preferred embodiment of the present invention the surface layer or first layer additionally comprises non-affine spacer molecules.

In a further preferred embodiment of the present invention the non-affine spacer molecules comprise a molecule selected from the group consisting of peptides, nucleic acids, dendrimers, dendrons, polyethylene glycol or a mixture thereof.

In another preferred embodiment of the present invention the linker comprises polyethylene glycol.

In yet further preferred embodiment of the present invention the mesh of non-affine spacer molecules is formed by the interaction of a multivalent binding molecule having a high affinity for a corresponding interaction partner.

In a further preferred embodiment of the present invention the multivalent binding molecule is a protein with multiple binding sites.

In a further preferred embodiment of the present invention the protein with multiple binding sites is streptavidin.

In a further preferred embodiment of the present invention the corresponding interaction partner is biotin.

In another preferred embodiment of the present invention the non-affine spacer molecule comprise polyethylene glycol.

In a further preferred embodiment of the present invention the length of said non affine spacer molecule is selected such that the resulting thickness of the shell structure is larger than the average roughness of the particle surface.

In another preferred embodiment of the present invention the length of said non affine spacer molecule is selected such that the electrostatic charge of said particles is considerably lowered.

In another aspect, the present invention relates to a particle, a flat structure or a matrix comprising such a coating, preferably comprising a material selected from the group consisting of agarose, polystyrene, latex, polyvinyl alcohol, silica and a magnetic material, wherein the first layer is coupled to the particle, flat structure, matrix, or nanoparticle surface.

In a further preferred embodiment of the present invention said particle is a nanoparticle.

In a further preferred embodiment of the present invention said particle, flat structure, or matrix comprising such a coating comprises a material selected from the group consisting of agarose, polystyrene, latex, polyvinyl alcohol, silica and a magnetic material.

In another preferred embodiment of the present invention the particle is a magnetic nanoparticle.

A further aspect the present invention relates to the use of such a coating, particle, flat structure, matrix, or nanoparticle for detection and/or purification of a specific target biomolecule from a sample and/or determination of the concentration of a specific target biomolecule.

In a further preferred embodiment of the present invention the specific target biomolecule is captured by the affinity molecule, wherein the specific target biomolecule is further recognized by a detection molecule.

In another preferred embodiment of the present invention the detection molecule is conjugated to an enzyme capable of generating a luminescent, fluorescent or electrochemical signal.

In a further preferred embodiment of the present invention the detection molecule is an antibody or a fragment thereof.

In a yet another preferred embodiment of the present invention the specific target biomolecule is detected via detection of clusters comprising at least two magnetic nanoparticles and at least one target biomolecule.

In an additionally preferred embodiment of the present invention the specific target biomolecule is detected via binding to a detection molecule on a sensor surface.

In another preferred embodiment of the present invention the purification of the target biomolecule comprises the steps of i) target biomolecule capture, ii) washing with an appropriate buffer, and iii) elution of the target biomolecule from matrix, surface or particle.

A further aspect of the present invention relates to the use of such a coating for i) improving the specificity of a preexisting surface layer comprising affinity molecules, and/or ii) preventing or minimizing non-specific binding to said preexisting surface layer, wherein the mesh of non affine spacer molecules is attached on top of a preexisting layer of affinity molecules.

Another aspect of the present invention relates to an assay for the detection of specific target biomolecules from a sample and/or determination of the concentration of specific target biomolecules comprising the use of such a coating, particle, flat structure or matrix, or nanoparticle.

In a preferred embodiment of the present invention the detection of said target biomolecules is performed in an optomagnetic system, wherein said magnetic nanoparticles are magnetically actuated and optically detected in a stationary sample fluid, comprising the steps of i) target biomolecule capture, ii) magnetic actuation, and iii) detection.

### BRIEF DESCRIPTION OF THE DRAWINGS

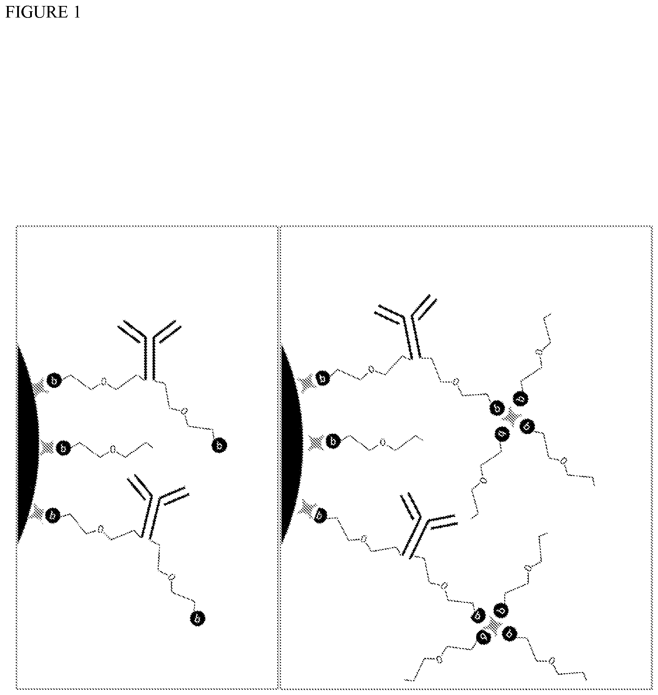

- Fig. 1: shows as schematic representation of a molecular two-layer architecture. In this Example, a streptavidin-coated particle is used as starting material. Streptavidin is indicated as a grey star. In the left panel a first layer is added to the particle. The first layer consists of a mixture of biotinylated antibodies and biotinylated spacer molecules. In the right panel a second layer is added to the first layer. The second layer comprises streptavidin bound to biotinylated spacer molecules. Streptavidin is multivalent, i.e. has more than one binding site for biotin.

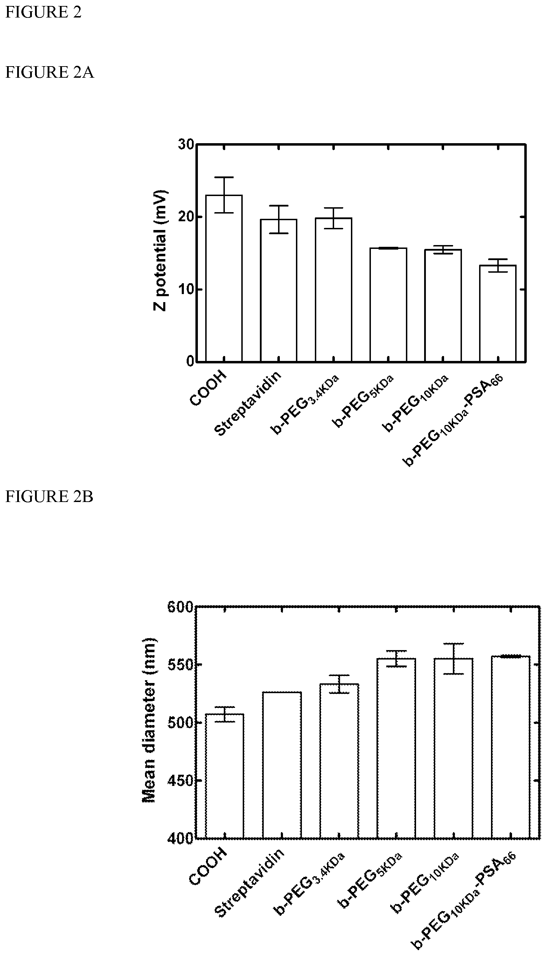

- Fig. 2: depicts diagrams showing zeta-potential (Fig. 2A) and mean diameter of nanoparticles (Fig. 2B) with different surface modifications. The shortest length of PEG that effectively prevents non-specific clustering of particles is approximately 12 nm (3.5 kDa).

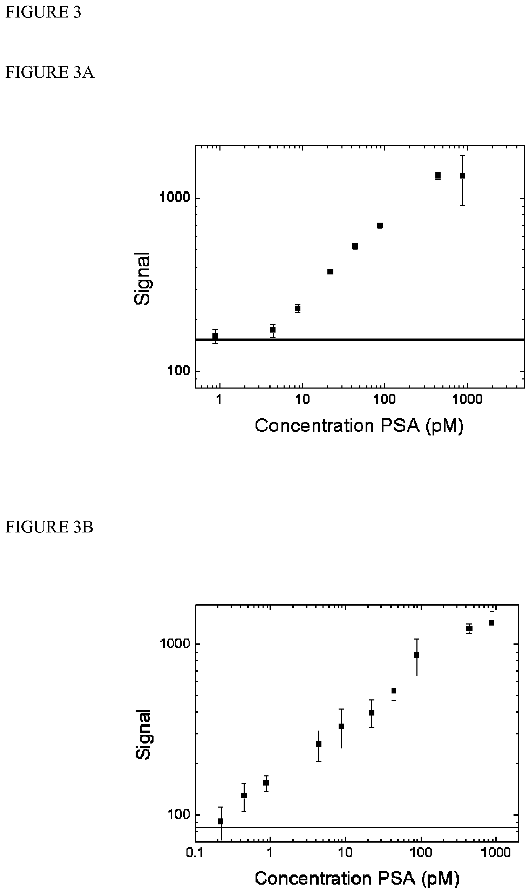

- Fig. 3: shows diagrams showing dose-response curves. The horizontal line represents the blank level (V/Hz^{0.5}) In Fig. 3A a dose-response curve of nanoparticles without a modified surface architecture is depicted. The limit of detection is approximately 5 pm. Fig. 3B shows the dose-response curve of nanoparticles having a two-layer shell structure. The background level is reduced by approximately a factor of 2 and the limit of detection is improved to a value of approximately 200 fM.

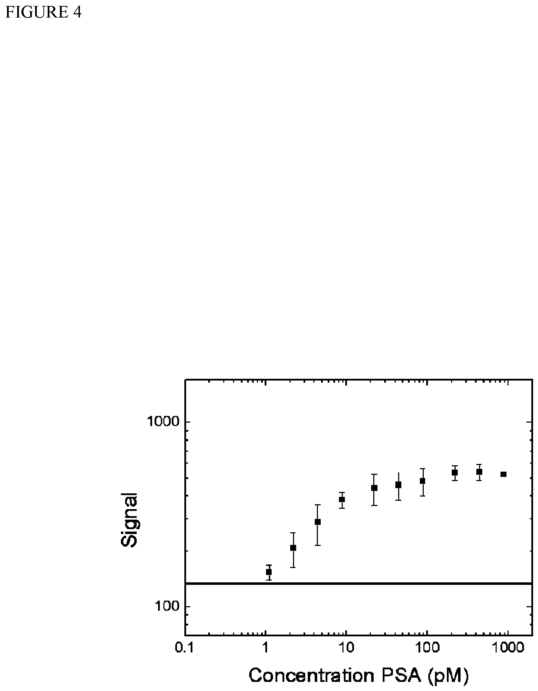

- Fig. 4: depicts a diagram showing the dose-response curve in 95% precleared human plasma pool. The modified surface results in a limit of detection of 0.7 pM. The early saturation is due to the higher viscosity of the complex matrix, which affects capture kinetics and critical frequency of the chemically-bound nanoclusters,

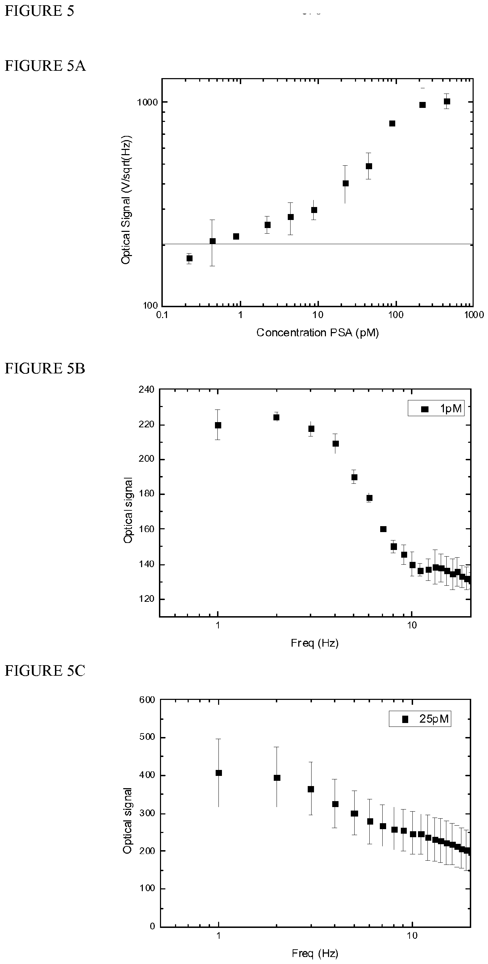

- Fig. 5: shows dose-response curves in untreated plasma pool from female donors. The limit of detection is approximately at 500 fM. The lower frequency dependent graphs (Fig. 5B and C) demonstrate size discrimination in plasma.

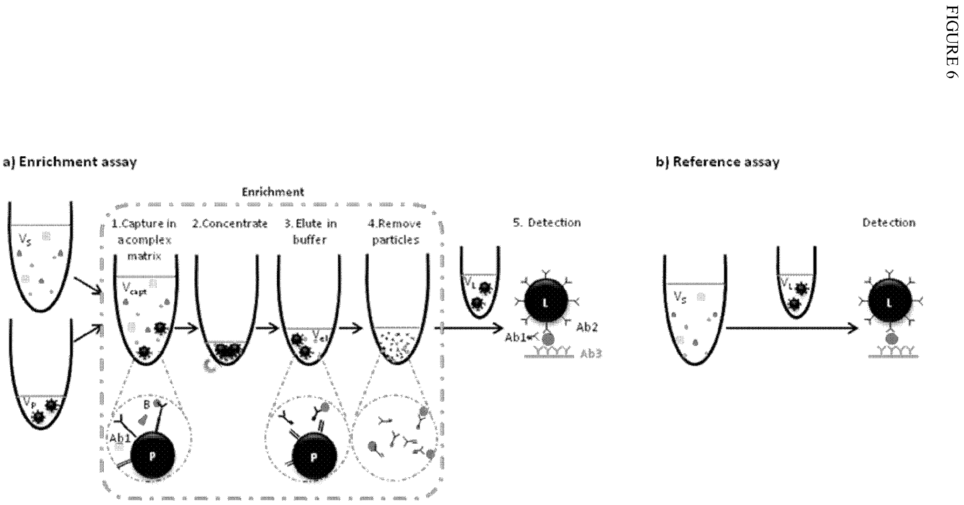

- Fig. 6: depicts an enrichment assay (panel a) and reference assay (panel b). Particles P coated with antibodies (Ab₁) capture the biomarker proteins (B). The particles are concentrated into a clean matrix of a smaller volume (Vₑ₁), wherein the capturing antibodies are eluted from the particles by enzymatic elution. The biomarkers are detected in a sandwich immunoassay in step 5. The biomarkers may be detected using an antibody coated sensor surface and antibody coated magnetic particles as labels (L). The labels may be detected by optical scattering in an f-TIR biosensor according to Bruls et al., 2009.

### DETAILED DESCRIPTION OF THE EMBODIMENTS

The present invention relates to the modification of surface structures for preventing non-specific binding.

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As has been set out above, the present invention concerns in one aspect a coating for preventing non-specific binding of molecules to a surface layer comprising an affinity molecule, said coating comprises a shell structure, wherein said shell structure comprises a first layer comprising one or more affinity molecules and further a second layer, which is coupled to the first layer, and wherein said second layer comprises non-affine spacer molecules forming a mesh.

The term "coating" as used herein refers to one or more layers of molecules spread over a surface for protection. Typically, coating as used herein may comprise one, two, three or more layers, which have the same or different composition.

The term "surface" as used herein refers to any surface type, surface structure or surface size. The term includes two and three dimensional surfaces and preferably includes any surface category or form known in the ambit of molecular interactions. Such surfaces may, in specific embodiments, have one or more activities, e.g. they may have a chemical, physical or biochemical/biological activity. A chemical activity may, for example be the capability of allowing chemical reactions in the form of a catalyst. A biological activity may, for example, be the capability of binding one or more biologically relevant entities or molecules, or performing enzymatic reactions etc. In specific embodiments, a surface may, for example, already comprise a coating, such as, for example, a coating according to the present invention. Alternatively or additionally, the surface maybe of structural importance or have structurally distinguishable elements, e.g. by providing planar, non-planar elements, by being planar or non-planar. It may further comprise one or more crates in a planar or quasi planar surface, one or more scratches in a planar or quasi planar surface, one or more holes in a planar or quasi planar surface, one or more embossments in a planar or quasi planar surface, one or more rims of material in a planar or quasi planar surface, it may have rough structure, or comprise one or more protrusions or protuberances.

A surface may have a certain degree of roughness, which is understood as a measure of the texture of a surface. A roughness of the surface may be quantified by vertical deviations of a real surface from its ideal form. If these deviations are large, the surface is considered rough; if they are small the surface is considered smooth. For the purpose of the present invention the roughness of the surface is considered to be the high frequency, short wavelength component of a measured surface. Measurements of the roughness may be carried out with any suitable technique or methodology, e.g. by determining profile roughness parameters, amplitude parameters and/or slope, spacing, and counting parameters. Typically, rough surfaces may have a higher friction coefficient than smooth surfaces.

One major function of the coating according to the present invention is to prevent or minimize non-specific binding to a surface, e.g. by preventing a biological activity of a surface as mentioned herein above such as in the case of biofouling. Coating thus means a covering layer, which may be used to cover or protect a preexisting surface layer of affinity molecules. For instance, if the coating is a single layer, the layer can also be constructed as to comprise mainly non-affine spacer molecules. For instance, such a single layer can be applied on surfaces, which already are coated with affinity molecules. The coating architecture may also be built in such a way that the affinity molecules are embedded within the coating structure, i.e. the affinity molecules constitute a part of the one or more coating layers.

One further important aspect is that the coating due to its molecular architecture may act as a steric barrier or spacer, which prevents binding of other molecules, or, which keeps other molecules or particles in a certain distance. The coating according to the present invention may also be construed such that they form a network or mesh of molecules. Therefore, a layer of a coating according to the present invention may be comprised of small units or entities, which have identical or similar chemical, physical and/or biological properties. Preferably, a layer of the coating as described herein may comprise biological or chemical molecules capable of forming polymers of a certain length. Suitable polymers are, for instance, nucleic acids, ribonucleic acids, peptides, proteins, carbohydrates, lipids, polyethylene glycol, polysaccharides, dendrimers, dendrons, nanotubes, or gold nanoparticles, or a mixture thereof. Preferably, these polymeric entities are comprised in linker- or non-affine spacer molecules. In specific embodiments of the present invention, the coating may be comprised of a single surface layer or shell structure.

The term "mesh of molecules" or "network of molecules" as used herein refers to a structure comprising building blocks and pores in the range between 0.3 nm and 1 micrometer, more preferably between 1 nm and 100 nm. The pore diameter may, for example be in a range of about 0.3 nm to about 400 nm, e.g. pores of a diameter of about 0.3 nm, 0.4 nm, 0.5 nm, 0.75 nm, 1 nm, 2 nm, 3 nm, 5 nm, 10 nm, 15 nm, 20 nm, 25 nm 30 nm, 35 nm, 40 nm, 50 nm, 60 nm, 70 nm, 80 nm, 90 nm, 100 nm or any suitable smaller or larger diameter.

Furthermore, such a mesh or network may have distinguished degrees of flexibility, which may depend on the size, amount, structure, and/or rigidity of the molecules present in said mesh or network. Accordingly, the flexibility may be adjusted by modifying any of the above mentioned parameters, as would be known to the person skilled in the art.

The term "single surface layer" as used herein refers to a single layer capable of forming a dense mesh or network of molecules. For example, the single surface layer may comprise one or more types of non-affine spacer molecules, preferably a single type of non-affine spacer molecules or a combination of compatible non-affine spacer molecules. The single surface layer may have a certain thickness, e.g. ranging from about 1 nm to 200 nm, preferably form about 3 nm to 150 nm, more preferably from about 5 nm to 120 nm, even more preferably from about 10 nm to about 100 nm. The thickness may vary in dependence on the molecules present, the mesh and network size and other factors known to the skilled person. For example, a single surface layer can be applied on top of a preexisting surface layer. In specific embodiments of the present invention, a single surface layer may constitute the outermost layer of a structure, e.g. a structure, which is composed of two or more internal layers. Such internal layers may differ structurally or chemically from the single surface layer. In further, specific embodiments of the present invention a single layer may comprise a layer of the thickness of about one non-affine spacer molecule, e.g. as depicted in Fig. 1 in the context of biotin linkers. The present invention is however not limited to this linker type, but encompasses further non-affine spacer molecules known to the person skilled in the art or suitable for the described approach.

The term "shell structure" as used herein refers an envelope like structure, which is typically comprised of small units or entities, which have identical or similar chemical, physical and/or biological properties. Preferably, the shell structure is comprised of two different or identical layers of molecules forming a double-layer or bilayer structure. Particular preferred are shell structures comprising a first and a second layer. The shell structure according to the present invention may cover any surface as mentioned herein above, for example the surface of solid forms, essentially flat surfaces, the surface of any particle, e.g. of a particle with spherical or ellipsoid shape, or the surface of any material, e.g. metal, organic material, inorganic material or any combination thereof with an irregular form such as a matrix. In further, specific embodiments of the present invention a shell structure may comprise a layer of the thickness of about two or three non-affine spacer molecules, e.g. as depicted in Fig. 1, right panel, in the context of biotin linkers. The present invention is however not limited to this linker or linker type, but encompasses further non-affine spacer molecules known to the person skilled in the art or suitable for the described approach.

The term "first layer" or "surface layer" refers to a layer of molecules, which is directly or indirectly bound to a surface structure, e.g. a surface as defined herein above. In specific embodiments of the present invention, the first layer or surface layer may comprise one or more affinity molecules. In a multi- or double-layered structure, the first layer may be the layer which is directly or indirectly coupled to the surface structure.

The term "directly bound to a surface structure" as used herein refers to a direct interaction between a layer molecule, e.g. a non-affine spacer molecule, and one or more surface molecules or a surface structure. Such an interaction may be based on covalent binding, van-der-Waals interactions, or electrostatic interactions.

The term "indirectly bound to a surface structure" as used herein refers to an indirect interaction via a binding molecule in between the non-affine spacer molecule and one or more surface molecules or a surface structure. The binding molecule in turn may be bound directly to one or more surface molecules or the surface structure.

The term "second layer" as used herein refers to a layer which may be identical to the first layer or which differ from the first layer, e.g. by the presence or absence of an affinity molecule, the identity or non-identity of the non-affine spacer molecule, its thickness etc. In specific embodiments of the present invention said second layer may not comprise an affinity molecule as defined herein. The second layer may, thus, in certain embodiments comprise one or more affinity molecules as defined herein. Alternatively, first and second layer may comprise an affinity molecule as defined herein.

In further embodiments of the present invention the shell structure may comprise more than two layers, e.g. 3, 4, 5, 6, 7, 8 or more layers as defined herein, e.g. constitute a multi -layer structure. These layers may be present in different configurations, e.g. a first layer as defined herein above may be covered by 2, 3, 4, 5, or more second layers as defined herein above. Alternatively, a first layer as defined herein above may be covered by further first layers as defined herein above. In further alternatives, a first layer as defined herein above may be covered by a second layer as defined herein above, followed by a further first layer etc, or may be further followed by a further second layer and/or first layer as defined herein above, etc. Further combinations of first and second layers are also envisaged by the present invention.

The term "coupled" as used for the conjunction of first and second layer refers to a covalent, or non-covalent binding between first and second layer elements. Such a coupling may for example a covalent binding, a van-der-Waals binding or a electrostatic binding between the layers. In specific embodiments, the binding between the layers may be reversible or may be terminated, e.g. by changing the pH, the temperature, the concentration of ions, by illuminating with light, by enzymatic degradation or enzymatic cleavage etc. such that, for example, one or more outermost layers are shed.

The term "affinity molecule" refers to any molecule having a high binding affinity for a second molecule, i.e. an interaction partner. Affinity molecule in the sense of the present invention typically comprise binding or capture molecules capable of binding a specific target molecule or capable of binding a molecule-containing target entity, such as for example a virus, or a cell or a cell fragment, or material derived from tissue. A target molecule may be any molecule bound by an affinity molecule. Preferably, a target molecule within the context of the present invention is a nucleic acid, e.g. DNA, or RNA molecule or an oligonucleotide such as a DNA or RNA oligonucleotide. Even more preferred are target molecules such as a peptide, a protein, a drug molecule, a small molecule, or a vitamin.

In a particularly preferred embodiment, the affinity molecule is selected from the group consisting of aptamers, peptides, proteins, oligonucleotides, and molecular imprinted polymers, wherein said affinity molecule preferably is an antibody or a fragment thereof.

An" aptamer" as used within the context of an affinity molecule may be a short nucleic acid molecule, e.g. an RNA, DNA, PNA, CNA, HNA, LNA or ANA molecule or any other suitable nucleic acid format known to the person skilled in the art, being capable of binding to a target molecule as defined herein, preferably to a nucleic acid target molecule as defined herein. Furthermore, the present invention envisages peptide aptamers, i.e. aptamers which are able to specifically bind to (a) protein(s), polypeptide(s) or peptide(s) comprising a specific amino acid sequence(s). Typically, (a) peptide aptamer(s) is/are a variable peptide loop(s), comprising for example 10 to 20 amino acids. In the context of the present invention the peptide aptamer(s) may in specific embodiments be attached at one or both ends to a scaffold structure. The scaffold structure may be any molecule, preferably a protein, e.g. a protein, which has good solubility properties. Suitable scaffold molecules would be known to the person skilled in the art. An example of suitable scaffold molecule to be used in the context of the present invention is the bacterial protein thioredoxin-A. The aptamer peptide loop may preferably be inserted within a reducing active site of the scaffold molecule. Alternatively, staphylococcal protein A and domains thereof and derivatives of these domains, such as protein Z or lipocalins may be used as scaffold structures in the context of the present invention. Nucleic acid or peptide aptamers may be generated according to any suitable method known to the person skilled in the art, e.g. via PCR or molecular synthesis approaches or yeast two-hybrid approaches.

A "peptide" as used within the context of an affinity molecule may comprise or alternatively consist of a stretch of 2 to 35 amino acids, amino acid derivatives or a mixture thereof. The peptide may be linear, branched, circular or mixture thereof. A peptide affinity molecule may also be attached to a scaffold structure as defined herein above.

A "protein" as used within the context of an affinity molecule may comprise or alternatively consist of a stretch more than about 35 amino acids, amino acid derivatives or a mixture thereof. The protein may have a linear, branched, circular form or be comprised of a mixture of these forms. A protein affinity molecule may also be attached to a scaffold structure as defined herein above.

An "oligonucleotide" as used within the context of an affinity molecule may comprise or alternatively consist of a stretch of about 5 to 120 nucleotides, e.g. a stretch of 10, 20, 30, 40, 50, 60, 70, 80, 90 or 100 nucleotides, preferably of about 15 to 60 nucleotides. An oligonucleotide affinity molecule may preferably be an RNA molecule or a DNA molecule, or a mixture of both.

The term "molecular imprinted polymer" as used herein refers to a polymer which was formed in the presence of a molecule that is extracted afterwards, leaving complementary cavities behind. Typically, a molecular imprinted polymer shows a certain chemical affinity for the original molecule. A molecular imprinted polymer may be composed of any suitable polymeric unit known to the person skilled in the art. Techniques for their production include polymerization techniques such as bulk, precipitation, emulsion, suspension, dispersion, gelation, and multi-step swelling polymerization. Particularly preferred are hierarchical imprinting methods.

An "antibody" as used within the context of an affinity molecule refers to an immunoglobulin molecules and immunologically active portions of immunoglobulin molecules, i. e. molecules that contain an antigen binding site that immunospecifically binds an antigen. The immunoglobulin molecules of the invention can be of any type (e. g., IgG, IgE, IgM, IgD, IgA and IgY), class (e. g., IgG1, IgG2, IgG3, 1gG4, 1gA1 and IgA2) or subclass of immunoglobulin molecules Antibodies of the present invention may be described or specified in terms of the epitope(s) or portion(s) of a polypeptide of the present invention which they recognize or specifically bind. Specific epitopes and their interaction with antibodies would be known to the person skilled in the art. The term "specifically binding" as used herein refers to the immunospecific detection and binding of an antibody to an antigenic epitope. The term "specifically binding" excludes non-specific binding but does not necessarily exclude cross-reactivity with other antigens, in particular with antigens comprising the same antigenic epitope detected by the present antibody.

In a preferred embodiment the antibody may be a polyclonal, monoclonal, multispecific, human, humanized or chimeric antibody, single chain antibody, or constitute a Fab fragment, Fab' fragment, a fragment produced by a Fab expression library, F(ab')2, Fv, disulfide linked Fv, minibody, diabody, scFv, sc(Fv)2, whole immunoglobulin molecule, small modular immunopharmaceutical (SMIP), binding-domain immunoglobulin fusion protein, camelized antibodiy, V_{HH} containing antibody, an anti-idiotypic (anti-Id) antibody an any epitope-binding fragment(s) of any of the above. Most preferably, the antibodies are human antigen-binding antibody fragments of the present invention and include Fab, Fab' and F (ab')2, Fv, single-chain Fvs (scFv), sc(Fv)2, single-chain antibodies, disulfide-linked Fvs (sdFv) and fragments comprising either a VL or VH domain.

The antibodies according to the invention may be from any animal origin including birds and mammals. Preferably, the antibodies are human, murine (e. g., mouse and rat), donkey, monkey, rabbit, goat, guinea pig, camel, horse, or chicken antibodies.

The antibodies according to the present invention may be monospecific, bispecific, trispecific or of greater multispecificity. Multispecific antibodies may be specific for different epitopes of a polypeptide of the present invention or may be specific for both a polypeptide of the present invention as well as for a heterologous epitope, such as a heterologous polypeptide or solid support material. Preferred are monospecific antibodies.

The term "non-affine spacer molecules" as used herein refers to molecules which primarily have the function of a spacer. For this purpose, these polymeric molecules have a certain length and strength in order to be able to act like polymeric fibers.

In specific embodiments of the present invention, the non-affine spacer molecules may have a length of up to about 500 nm, preferably up to about 450, 400, 350, 300, and 250 nm, even more preferably of up to 200, 150, 100, 90, 80, 70, 60, 50, 40, 30, 20, and 10 nm.

In further specific embodiments of the present invention, the non-affine spacer molecules may be linear, circular, or branched-like molecules, or a mixture thereof. If they are branched, they may be branched in different degrees, e.g. show 2, 3, 4, 4 or more hierarchical levels. The present invention further envisages a combination of different types, e.g. linear and branched, different branching degrees, different spacer lengths etc. of non-affine spacer molecules within a network, layer or shell structure. In alternative embodiments of the present invention the spacer molecule or mixture of spacer molecules provide an uniform mesh or network, i.e. an equispaced mesh or network with openings of essentially the same size. It is particularly preferred that the mesh be provided with openings allowing that target molecules in solution to effectively approach an affinity molecule inside the mesh, in particular the binding regions (e.g. the paratopes) of these affinity molecules. In addition, the affinity molecules should preferably have some motional freedom, so that they are functional for binding to the targets. It is in particular preferable if the mesh is so uniform, so that the functional properties of the mesh with respect to the approachability of affinity molecules by target molecules and the motional freedom of affinity molecules are uniform. These parameters may be adjusted by the lengths, branching degree etc. of the non-affine spacer molecule as defined herein.

In further specific embodiments of the present invention, the non-affine spacer molecules can directly or indirectly be interlinked so as to form a three dimensional mesh of molecules. Such an interlinking may be based on functional groups at the termini of a molecule, or located in the center of a molecule, e.g. in case branching is envisaged. The control of interlinkage, its degree etc. may be implemented according to principles and methods known to the person skilled in the art, e.g. from a qualified textbook such as "Bioconjugate techniques", Hermanson, 2nd Ed. Academic Press, Elsevier or "Dendrimers and other dendritic polymers" Frechet and Tomalia, J. Wiley.

The term "non-affine" as used herein refers to the capability of the spacer molecule as being non binding to other molecules or entities or as being not bound by other molecules or entities. For example, the non-affine spacer molecule may be chemically inert, lack functional or reactive groups, be unable to bind to biological molecules such as proteins, peptides, nucleic acids, or derivatives thereof. In specific embodiments of the present invention these non-affine spacer molecules may, however, be able to mechanically restrain other molecules or entities, e.g. in a small mesh or closed mesh. Such withholding capability is typically due to structural properties of the mesh or network, not due to molecule-molecule interactions.

Suitable non-affine spacer molecules herein disclosed may be, for example, nucleic acids, ribonucleic acids, peptides, proteins, carbohydrates, lipids, polyethylene glycol, polysaccharides, dendrimers, dendrons, nanotubes, or gold nanoparticles, or any suitable derivatives or combinations thereof. The group of non-affine spacer molecules may specifically comprise hydrocarbon-based surfactants, cholesterol, glycolipids, bile acids, saponins, fatty acids, synthetic amphipathic block copolymers, natural products like egg yolk phospholipids.

"Nucleic acid non-affine spacer molecules" may be any nucleic acid molecules which are not provoking a specific molecular binding reaction, in particular no molecular binding reaction interfering with an affinity molecule as defined herein. Such molecules may comprise single and/or double stranded DNA molecules, or any type of derivative thereof. The molecule may also be a PNA, CNA, HNA, LNA or ANA molecule, or any mixture or combination thereof, or any mixture or combination with any other non-affine spacer molecule as defined herein. In specific embodiments, the non-affine spacer molecule is a ribonucleic acid molecule, or any mixture of a ribonucleic acid with any of the other mentioned nucleic acid molecules or with any other non-affine spacer molecule as defined herein. The nucleic acid or ribonucleic acid molecules may be of different base compositions and/or lengths. The length of the molecule may vary between about 5 nucleotides to about 5000 nucleotides. Also envisaged are other lengths or any length value within the indicated range. The length may be made dependent on the envisaged mesh size or pore size, the envisaged size of the layer(s) and/or the size or amount of affinity molecules present in the layer or shell structure, and/or the envisages flexibility or stability of the layer or shell structure.

Preferred nucleic acids are single stranded DNA molecules of different base compositions and/or lengths. The single stranded molecules may, for example, encompass the repetition of base sequence, be entirely random, or be derived from nature, or be of only one base, e.g. AAA etc., TTT etc., CCC etc., or GGG etc.; or comprise stretches of such monobase regions, e.g. be comprised only of A and C, or C and T.

In specific embodiments of the present invention the nucleic acid non-affine spacer molecules may be able to hybridize or be hybridized to complementary nucleic acid molecules, or preferably to partially complementary nucleic acid molecules. The region of complementarity may comprise, for example, about 5 to 65% of the molecule. Thereby an adjustment of the size, flexibility, degree of meshing or of the pore sizes may be possible.

"Peptide non-affine spacer molecules" may be any peptide molecules which are not provoking a specific molecular binding reaction, in particular no molecular binding reaction interfering with an affinity molecule as defined herein. Such peptides may be of different amino acid compositions and/or lengths. The length of the molecule may vary between about 3 amino acids to about 35 amino acids. Also envisaged are other lengths or any length value within the indicated range. The length may be made dependent on the envisaged mesh size or pore size, the envisaged size of the layer(s) and/or the size or amount of affinity molecules present in the layer or shell structure, and/or the envisaged flexibility or stability of the layer or shell structure. The amino acid composition may vary between mono amino acid compositions, e.g. only one of the naturally produced amino acids or any derivative thereof or of synthetically available amino acids, and completely random amino acid compositions comprising or be selected from all known amino acids. Also envisaged are compositions comprising 2, 3, 4, 5, 6, 7, 8, 9, 10, 11, 12, 13, 14, or 15 different amino acids. The amino acids may be present in stretches of identical amino acids or be provided in the form of pattern or motifs, or be randomly distributed.

"Protein non-affine spacer molecules" may be any protein molecules which are not provoking a specific molecular binding reaction, in particular no molecular binding reaction interfering with an affinity molecule as defined herein. Such proteins may be of different amino acid compositions and/or lengths. The length of the molecule may vary between about 35 amino acids to about 500 amino acids, or more. Also envisaged are other lengths or any length value within the indicated range. The length may be made dependent on the envisaged mesh size or pore size, the envisaged size of the layer(s) and/or the size or amount of affinity molecules present in the layer or shell structure, and/or the envisaged flexibility or stability of the layer or shell structure. The amino acid composition may vary between mono amino acid compositions, e.g. only one of the naturally produced amino acids or any derivative thereof or of synthetically available amino acids, and completely random amino acid compositions comprising or be selected from all known amino acids. Also envisaged are compositions comprising 2, 3, 4, 5, 6, 7, 8, 9, 10, 11, 12, 13, 14, or 15 different amino acids. The amino acids may be present in stretches of identical amino acids or be provided in the form of pattern or motifs, or be randomly distributed.

"Carbohydrate non-affine spacer molecules" may be any carbohydrate molecules which are not provoking a specific molecular binding reaction, in particular no molecular binding reaction interfering with an affinity molecule as defined herein. Such carbohydrates may be of different compositions and/or lengths. The length of the molecule may vary between about 5 C atoms to about 100 C atoms, or more. Also envisaged are other lengths or any length value within the indicated range. The length may be made dependent on the envisaged mesh size or pore size, the envisaged size of the layer(s) and/or the size or amount of affinity molecules present in the layer or shell structure, and/or the envisaged flexibility or stability of the layer or shell structure.

"Lipid non-affine spacer molecules" may be any lipid molecules which are not provoking a specific molecular binding reaction, in particular no molecular binding reaction interfering with an affinity molecule as defined herein. Such lipids may be of different compositions and/or lengths. The length of the molecule may vary between about 5 C atoms to about 100 C atoms, or more. Also envisaged are other lengths or any length value within the indicated range. The length may be made dependent on the envisaged mesh size or pore size, the envisaged size of the layer(s) and/or the size or amount of affinity molecules present in the layer or shell structure, and/or the envisaged flexibility or stability of the layer or shell structure. Preferred lipids are phospholipids, e.g. phosphatidylethanolamine, phosphatidylcholine, egg phosphatidyl-ethanolamine, dioleoylphosphatidylethanolamine. Particularly preferred are the phospholipids MPPC, DPPC, DPPE-PEG2000 or Liss Rhod PE.

"Polyethylene glycol non-affine spacer molecules" may be any polyethylene glycol molecules which are not provoking a specific molecular binding reaction, in particular no molecular binding reaction interfering with an affinity molecule as defined herein. Such polyethylene glycols may be of different compositions and/or lengths. The length may be made dependent on the envisaged mesh size or pore size, the envisaged size of the layer(s) and/or the size or amount of affinity molecules present in the layer or shell structure, and/or the envisaged flexibility or stability of the layer or shell structure. Preferred polyethylene glycol (PEG) variants include polydispers or monodisperse PEG. Particularly preferred is monodisperse PEG. PEGs may further be branched, e.g. having 3-10 PGE chains emanating from a central core group, be star PEGs having 10 to 100 PEG chains emanating from a central core group, or be comb PEGs which have multiple PEG chains grafted to a different polymer or linear PEG backbone. According to the PEG's average molecular weights, envisaged PEGs maybe PEG 400, PEG 600, PEG 800, PEG 1000, PEG 1500, PEG 2000, PEG 3350, PEG 4000, PEG 6000, PEG 8000, PEG 10,000 or PEGs having higher molecular weights. Particularly preferred is PEG 10,000.

"Dendrimers" or "dendrimetic non-affine spacer molecules" may be any repeatedly branched, roughly spherical large molecules which are not provoking a specific molecular binding reaction, in particular no molecular binding reaction interfering with an affinity molecule as defined herein. Such dendrimers may be of different compositions and/or lengths, and/or degree of branching. The length and degree of braching may be made dependent on the envisaged mesh size or pore size, the envisaged size of the layer(s) and/or the size or amount of affinity molecules present in the layer or shell structure, and/or the envisaged flexibility or stability of the layer or shell structure. Dendrimers as envisaged by the present invention may comprise low-molecular weight or high-molecular weight species. Dendrimers according to the present invention may, in certain embodiments, comprise functional groups on the molecular surface, e.g. hydrophilic groups, or alternatively have internal functionality. Dendrimers as envisaged for the purposes of the present invention may be dendrimers of 1^{st}, 2^{nd}, 3^{rd}, 4^{th} or a higher generation. Preferred dendrimers include, for example, newkome dendrimer or arbolol, and Polyamidoamine (PAMAM). Further variants to be used within the context of the present invention, as well as synthesis methods etc. would be known to the person skilled in the art or can be derived from suitable documents, such as "Dendrimers and other dendritic polymers" Frechet and Tomalia, J. Wiley.

"Dendrons" or" dendronic non-affine spacer molecules" are understood as containing a single chemically addressable groups of dendrimers, i.e. a focal point of a dendrimer as defined herein above.

The term "nanotube" as used herein refers to carbon nanotubes, i.e. allotropes of carbon with a cylindrical nanostructure. Such nanotubes may be single walled nanotubes or multi walled nanotubes. The present invention also envisages spacer molecules of the fullerene structural family of different types, forms and complexity, e.g. spherical, or ellipsoid buckyball forms, buckyball fullerene, or a combination of nanotubes with backbyballs etc.

In the present invention the above defined non-affine spacer molecules are provided with an interactor functionality, e.g. a chemical group or residue allowing an interaction with other molecules, e.g. with other non-affine spacer molecules, and/or with one or more binding molecules, and/or with one or more affinity molecules as defined herein above. Preferred is the employment of a biotin or biotin-like functionality, which is fused or linked to a non-affine spacer molecule as defined herein above. The fusion may be at one or more termini of the spacer molecules, or be carried out within the molecule(s).

It is particularly preferred that a mesh according to the present invention, or a coating comprising such a mesh, or a single or multiple layer structure as defined herein above or a shell structure as defined herein above comprises an affinity molecule, e.g. an antibody in a high density. It is additionally or alternatively preferred that said affinity molecule, e.g. antibody be in a molecular conformation that is effective for specific binding of target molecules, e.g. of specific antigens or epitopes thereon. It is additionally or alternatively preferred that affinity molecules be outward orientied form the particle core, in particular from a magnetic particle, such as a magnetic nanoparticle. It furthermore additionally or alternatively preferred that an affinity molecule according to the present invention be mobile, e.g. sufficiently mobile in order to reach out for target molecules and form biochemical bonds.

In an illustrative example and particularly preferred embodiment of the present invention these features have been implemented on particles comprising streptavidin and biotin-coupled PEG spacers of a range higher than about 3.5 kDa, or a length of about 40 nm together with an anti-PSA antibody. The present invention is however, not limited to this conjunction of parameters, but encompasses further variants as described herein above or below.

In preferred embodiments of the present invention a surface layer or first surface as defined may be coupled to a particle surface or to a flat surface or to matrix or matrix like structure. The term "particle surface" as used herein refers to the form of the entity on which the surface is located as being a small localized object to which can be ascribed a physical property such as volume or mass. Furthermore, a particle essentially behaves as a whole unit in terms of its transport and properties. Particles and hence their surfaces may accordingly be of a symmetrical, globular, essentially globular or spherical shape, or be of a irregular, asymmetric shape or form. The particle size (and hence the dimension of the surface) may vary. Preferred are particles and particle surfaces in the nanometer and micrometer range up to several micrometers. Particularly preferred are surfaces of nanoparticles, e.g. of particles of a diameter of about 1 to 700 nanometers. Particularly preferred are surfaces of nanoparticles which may have a diameter of about 10 to 600 nm, e.g. 10 nm, 15 nm, 20 nm, 25 nm, 30 nm, 35 nm, 40 nm, 45 nm, 50 nm, 60 nm, 70 nm, 80 nm, 90 nm, 100 nm, 120 nm, 150 nm, 170 nm, 200 nm, 220 nm, 250 nm, 270 nm, 300 nm, 320 nm, 350 nm, 370 nm, 400 nm, 420 nm, 450 nm, 470 nm, 500 nm, 520 nm, 550 nm, 570 nm, 600 nm, 620 nm or any value in between. Even more preferred are nanoparticles having a diameter of about 500 nm.

A "flat surface" as used herein refers to planar, essentially planar or quasi-planar surfaces, e.g. present in or on devices or larger, non particulate objects or entities. Such surfaces may also, in specific embodiments, include inclinations, protrusions, irregularities etc. Further embodiments envisage the combination of flat surfaces in different directions, in different angles, e.g. on geometric entity or objects such as a cube or cuboid structure.

A "matrix" as used herein refers to an irregularly shaped structure, which comprises cavity or hole elements. In specific embodiments, such cavities or holes may be present on a solid body of substance or underlying solid layer. In other embodiments, the entire object may be comprised of cavities or holes, e.g. in the form of a mesh or 3 dimensional lattice. The surface of matrix entities may accordingly be inclined, non-planar with frequent changes or inclination.

In further preferred embodiments of the present invention the material of the object on which the above mentioned surface is located may be specific. For example, the material may be of inorganic or organic nature. For example, the material may comprise, essentially consist of or consist of a metal or an alloy of metals, or an organic material comprising carbohydrate elements. Examples of envisaged material include agarose, polystyrene, latex, polyvinyl alcohol, silica and a ferromagnetic metals, alloys or composition materials. Particularly preferred are magnetic or ferromagnetic metals, alloys or compositions.

In further preferred embodiments, the material may have specific properties. The material may, for example, be magnetic or be non-magnetic. The material may, in other embodiments, be hydrophobic, or hydrophilic.

In a particularly preferred embodiment, the material is a magnetic material. In further particularly preferred embodiments, the entity on which a surface as defined herein above is present, or on which a coating according to the present invention may be placed is a magnetic nanoparticle.

A "binding molecule" as used herein is to be understood as a connector in between a non-affine spacer molecule as defined herein and one or more surface molecules or a surface structure as defined herein, and/or as a connector between two or more non-affine spacer molecules as defined herein. A binding molecule may, in specific embodiments, be monovalent, bivalent, trivalent or multivalent for identical or similar interactions or their capability of interactions, i.e. a binding molecule may bind one, 2, 3, or more identical interactor molecules, e.g. non-affine spacer molecules or surface entities. Alternatively, or additionally, the binding molecule may be bivalent, trivalent or multivalent with respect to different interactor molecules, e.g. non-affine spacer molecules or surface entities. Thus, a binding molecule may bind one, two, 3, 4, 5, 6, 7 or more different interactor molecules, e.g. non-affine spacer molecules or surface entities. Also a combination of both capabilities may be present within one binding molecule, i.e. binding molecule may bind two or more identical interactors and at the same time one, two, three or more different interactors etc. In a preferred embodiment a binding molecule may be protein or peptide, e.g. a protein with multiple binding sites. A particularly preferred example would be streptavidin. Alternatively, binding molecules such as avidin, streptavidin derivatives, (strept)avidin, avidin-related proteins, avidin-like entities such as tamavidin 1 and 2, bradavidin, NeutrAvidin etc. may be used.

In a further particularly preferred embodiment an non-affine spacer molecule such as polyethylene glycol may be provided with a interactor functionality such as biotin, being capable of binding to a binding protein such as streptavidin. The present invention such envisages in a particularly preferred embodiment a first layer or shell structure essentially comprising PEG, biotin and streptavidin as structural components. The layer or shell structure may additionally comprise affinity molecules such as preferably an antibody as defined herein above.

In a further specific embodiment of the present invention the coating as defined herein above, e.g. comprising a shell structure or a single layer as defined herein, may be devised according to or in dependence of the length of a non-affine spacer molecule as defined herein above. For example, a shell structure may comprise one or two layers if the non-affine spacer is a long molecule comprising more than 20, 30, 40, 50, 60, 70, 80, 90, 100, 150, 200, 300, 400, 500, 600, 700 or 1000 single units (C-atoms, nucleotides, amino acids, functional groups etc.) as described herein.

In further embodiments of the present invention the length of a non-affine spacer molecule may be selected in accordance with or in dependence of the surface of an entity, in particular of a particle. Preferably, the length of non-affine spacer molecules may be selected such that the resulting thickness of the shell or layer structure or coating is larger than the average roughness of an object's surface, as defined herein above, e.g. determined by profile roughness parameters, amplitude parameters and/or slope, spacing, and counting parameters. In a preferred embodiment, the length of non-affine spacer molecules may be selected such that the resulting thickness of the shell or layer structure or coating is larger than the average roughness of a particle's surface, more preferably of a nanoparticle's surface as defined herein above, e.g. determined by parameters as mentioned above. As herein described the length of a non-affine spacer molecule may be selected in accordance with or in dependence of the electrostatic charge of an object, e.g. of a particle, preferably of a nanoparticle. Preferably, the length of non-affine spacer molecules may be selected such that the electrostatic charge of an object, particle, preferably a nanoparticle is lowered. The term "lowered" as used herein refers to a comparison between an object, in particular a particle with and without the coating, with and without a single layer o a layer of a multilayer structure, or with and without a shell structure as defined herein. If the electrostatic charge of the object, preferably the particle, with the coating is reduced by 10%, 20%, 30%, 40%, 50%, 60%, 70%, 80%, 100% or more than 100%, or a reduction by the factor of 2, 3, 4, 5, 6,7, 8,9 10, 20, 50, 100 or more takes place, the electrostatic charge may be considered as being lowered. It is preferred that the electrostatic charge be considerably lowered, e.g. by at least 50%. As herein described, if the electrostatic charge of an object, particle, preferably a nanoparticle is lowered as defined above, or alternatively, if said electrostatic charge of an object, particle, e.g. nanoparticle is identical (in comparison to an object or particle without coating as defined above) said object or particle, e.g. nanoparticle, may show a reduced non-specific clustering due to the presence of the layer structure, shell structure or coating as defined herein above. The term "reduced" as used in the context of the non-specific clustering means a reduction 10%, 20%, 30%, 40%, 50%, 60%, 70%, 80%, 100% or more than 100%, or a reduction by the factor of 2, 3, 4, 5, 6,7, 8,9 10, 20, 50, 100 or more. The reduction of the non-specific clustering may preferably be adjusted by the length of the non-affine spacer molecule(s). Alternatively, by tailoring the non-affine spacer molecule(s), e.g. by adjusted their length, the charge may also be also increased, which could further improve the degree of suppression on non-specific interactions. The term "increased" as used in the context of the non-specific interactions means an increase of 10%, 20%, 30%, 40%, 50%, 60%, 70%, 80%, 100% or more than 100%. As herein described the length of the non-affine spacer molecule may be selected such that the resulting thickness of the shell or layer structure or coating is larger than the average roughness of a particle's surface, more preferably of a nanoparticle's surface as defined herein above, e.g. determined by parameters as mentioned above and that the electrostatic charge of the particle, preferably nanoparticle be considerably lowered.

In a further aspect a coating, or layer structure as defined herein above may be used for the improvement of the specificity of preexisting surface layers comprising affinity molecules, wherein a mesh of non-affine spacer molecules is attached on top of a preexisting layer of affinity molecules. Preferably, second layer elements comprising non-affine spacer molecules and optionally binding molecules may be used to cover particles, structures or matrices comprising an affinity molecule as defined herein above, e.g. an antibody. Thereby, a two or multilayer structure or shell structure may be generated which largely corresponds to a two or multilayer structure or shell structure as defined herein above. Connection points or attachment points may be found either on the particle or object surface itself, or on the affinity molecules, e.g. antibodies (see also Fig. 1, right hand panel, which is considered as a specific embodiment of this approach, if starting from a situation as depicted in Fig. 1, left hand panel, or a similar situation where no non-affine spacer molecules are present).

In yet another aspect a coating, or layer structure as defined herein above may be used for preventing or minimizing non-specific binding to a preexisting surface layer, wherein a mesh of non-affine spacer molecules is attached on top of a preexisting layer of affinity molecules. Preferably, second layer elements comprising non-affine spacer molecules and optionally binding molecules may be used to cover particles, structures or matrices comprising an affinity molecule as defined herein above, e.g. an antibody. Thereby, a two or multilayer structure or shell structure may be generated which largely corresponds to a two or multilayer structure or shell structure as defined herein above. Connection points or attachment points may be found either on the particle or object surface itself, or on the affinity molecules, e.g. antibodies (see also Fig. 1, right hand panel, which is considered as a specific embodiment of this approach, if starting from a situation as depicted in Fig. 1, left hand panel, or a similar situation where no non-affine spacer molecules are present).

By providing a mesh structure around the affinity molecules, the unspecific binding of molecules or entities to an affinity molecule or to molecules in its vicinity may be reduced, or completely abrogated or avoided.

In a further aspect the present invention relates to a particle comprising a coating, single layer, multi-layer or shell structure as defined herein above. The present invention also relates to a flat structure comprising a coating, single layer, multi-layer or shell structure as defined herein above. A "particle" as used herein means a small, localized object to which can be ascribed a physical property such as volume or mass. Furthermore, a particle essentially behaves as a whole unit in terms of its transport and properties. Particles may accordingly be of a symmetrical, globular, essentially globular or spherical shape, or be of a irregular, asymmetric shape or form. The size of a particle envisaged by the present invention may vary. Preferred are particles in the nanometer and micrometer range up to several micrometers. Particularly preferred are nanoparticles, e.g. particles of a diameter of about 1 to 700 nanometers. Particularly preferred are surfaces of nanoparticles which may have a diameter of about 10 to 600 nm, e.g. 10 nm, 15 nm, 20 nm, 25 nm, 30 nm, 35 nm, 40 nm, 45 nm, 50 nm, 60 nm, 70 nm, 80 nm, 90 nm, 100 nm, 120 nm, 150 nm, 170 nm, 200 nm, 220 nm, 250 nm, 270 nm, 300 nm, 320 nm, 350 nm, 370 nm, 400 nm, 420 nm, 450 nm, 470 nm, 500 nm, 520 nm, 550 nm, 570 nm, 600 nm, 620 nm or any value in between. Even more preferred are nanoparticles having a diameter of about 500 nm.

A "flat structure" as used herein refers to planar, essentially planar or quasi-planar object, e.g. present in or on a device or apparatus, or on larger, non particulate entities or objects. Such flat structures may also, in specific embodiments, include inclinations, protrusions, irregularities etc. Further embodiments envisage the combination of flat structures being combined in different directions, in different angles, e.g. on geometric objects such as a cube or cuboid structure etc.

In a further aspect the present invention relates to a matrix as defined herein above comprising a coating, single layer, multi-layer or shell structure as defined herein above. A matrix may accordingly be coated such that any holes, irregularities or cavities be kept, or alternatively be partially, largely or completely closed. A matrix may thus, in specific embodiments, be converted upon the provision of a coating, layer, or shell structure according to the present invention as a flat or planar object or particle as defined herein above.

Particles, flat structures or matrices as defined herein above may comprise or consist of any suitable material known to the person skilled in the art, e.g. they may comprise or consist of or essentially consist of inorganic or organic material. Typically, they may comprise or consist of or essentially consist of metal or an alloy of metals, or an organic material, or comprise or consist of or essentially consist of carbohydrate elements. Examples of envisaged material include agarose, polystyrene, latex, polyvinyl alcohol, silica and ferromagnetic metals, alloys or composition materials. Particularly preferred are magnetic or ferromagnetic metals, alloys or compositions. In further preferred embodiments, the material may have specific properties. The material may, for example, be magnetic or be non-magnetic. The material may, in other embodiments, be hydrophobic, or hydrophilic.

Particularly preferred are magnetic particles comprising a coating, single layer, multi-layer or shell structure as defined herein above. Even more preferred are magnetic nanoparticles comprising a coating, single layer, multi-layer or shell structure as defined herein above.

In further preferred embodiments, the material may have specific properties. The material may, for example, be magnetic or be non-magnetic. The material may, in other embodiments, be hydrophobic, or hydrophilic. In particularly preferred embodiments of the present invention, the material, or particle, e.g. nanoparticle may be superparamagnetic particles, which are typically dispersed in aqueous solutions and retain a small negative charge keeping the particles separated and avoiding non-specific clustering.

In a particularly preferred embodiment, the material is a magnetic material. In further particularly preferred embodiments, the entity on which a surface as defined herein above is present, or on which a coating according to the present invention may be placed is a magnetic nanoparticle.

In further embodiments of the present invention, particles, flat structures or matrices as defined herein above, i.e. comprising a coating, layer structure or shell structure as defined herein above may be used for molecular diagnostics, biological sample analysis, chemical sample analysis, food analysis, and/or forensic analysis.

In another aspect the present invention refers to the use of a coating as defined herein above, e.g. of a single layer, or multiple-layer coating, or of shell structure as defined herein above, the use of a particle as defined herein above, the use of a flat structure as defined herein above, the use of a matrix as defined herein above, or the use of a nanoparticle as defined herein above for the detection of a specific target biomolecule from a sample.

The term "detection" as used herein refers to the specific recognition of a target molecule as defined herein above, preferably of a protein, drug molecule or nucleic acid. This specific recognition is preferably performed with the help of an affinity molecule as defined herein. A recognition may thus comprise a specific reversal or irreversible binding of a target molecule to an affinity molecule within a coating, single layer, or shell structure as defined herein above. This binding is subsequently visualized or recognized by any suitable means known to the person skilled in the art.

The detection may be carried out in any suitable environment. Example of such environments include a solution comprising a particle, structure, matrix or any other object comprising a coating as defined herein above. For example, a nanoparticle comprising a coating according to the present invention may be immersed or partially immersed in a solution. The solution may, for example, comprise a target molecule, e.g. in the form of a sample or processed sample. Additionally or alternatively, the solution may comprise one or more entities necessary for performing a detection as described herein above or below. In further embodiments of the present invention the detection may be carried out at a sensor surface or sensing surface, e.g. at the surface of a biosensor comprising magnetic detection means. Examples of such sensors which may be used in the context of the present invention are provided in Bruls et al., 2009, Lab Chip, 9, 3504-3510.

A "sample" as used herein refers to any sample, which includes a target molecules as defined herein. Such samples may, for example, include samples derived from or comprising stool, whole blood, serum, plasma, tears, saliva, nasal fluid, sputum, ear fluid, genital fluid, breast fluid, milk, colostrum, placental fluid, amniotic fluid, perspirate, synovial fluid, ascites fluid, cerebrospinal fluid, bile, gastric fluid, aqueous humor, vitreous humor, gastrointestinal fluid, exudate, transudate, pleural fluid, pericardial fluid, semen, upper airway fluid, peritoneal fluid, fluid harvested from a site of an immune response, fluid harvested from a pooled collection site, bronchial lavage, urine, biopsy material, e.g. from all suitable organs, e.g. the lung, the muscle, brain, liver, skin, pancreas, stomach, etc., a nucleated cell sample, a fluid associated with a mucosal surface, hair, or skin. In addition, samples from environmental sources, e.g. water samples, meat or poultry samples, samples from sources of potential contamination etc. may be used.

In some embodiments, target molecules may be directly obtained from samples. In other situations samples may be subjected to sample preparation techniques, e.g. based on standard protocols, including, for example, partial purification, which renders the target molecules more accessible to binding partners, i.e. an affinity molecule as defined herein. For example, blood samples may be centrifuged to separate fractions including whole cells or membranes from serum, feces samples may be sectioned and homogenized with physiologically acceptable buffer and detergent, sputum samples may be liquefied and fractionated. Furthermore, antibiotics or bactericides may be added to samples to prevent further growth of any organisms present. Whole cells may also be removed or may be lysed to release their contents.

Preferably, for the preparation of nucleic acid containing samples, inhibitors of DNA and RNA degrading enzymes and proteinases may be added. Furthermore, nucleic acid target molecules may also be amplified prior to detection, e.g. via suitable PCR reactions or ligase chain reactions as would be known to the person skilled in the art.

In further embodiments, nucleic acids in samples may be sheared or cut into smaller fragments (e.g., by mechanical shearing or restriction enzyme digestion). Preferred is a shearing process leading to nucleic acid molecules of a size of 50 to 500 nucleotides, preferably of 200 nucleotides. Typically, a shearing technique by focused ultrasound may be used, e.g. based on any suitable apparatus such as an instrument marketed by Covaris. Heterogeneous samples may further be purified to remove substantially all non-nucleic acid molecules prior to loading the sample into the device. Additionally, or alternatively, heterogeneous samples may be processed by separation techniques such as capillary electrophoresis, e.g. in order to deselect molecules outside of a desired length range, preferably molecules having a size outside of a range of 50 to 500 nucleotides.

In a particularly preferred embodiment the present invention relates to the use of a coating as defined herein above, e.g. of a single layer, or multiple-layer coating, or of shell structure as defined herein above, the use of a particle as defined herein above, the use of a flat structure as defined herein above, the use of a matrix as defined herein above, or the use of a nanoparticle as defined herein above for the detection of a specific target molecule, e.g. biomolecule, from a sample, wherein the specific target molecule is captured by an affinity molecule as defined herein above, and wherein said specific target molecule is subsequently recognized by a detection molecule. A "detection molecule" as used herein is any molecule suitable for recognizing an interaction between an affinity molecule as defined herein above and a specific target molecule bound to said affinity molecule. The detection molecule may, for example, recognize the target molecule specifically, and/or the interaction between the target molecule and the affinity molecule. Preferred examples of such detection molecules are antibodies or antibody fragments or variants as defined herein above, which specifically bind a target molecule which is captured or immobilized on an affinity molecule. In the case of antibody detection molecules, the antibodies may bind to different epitope of a target molecule than the affinity molecule, or may detect an epitope generated by a specific binding of the target molecule to an affinity molecule. Furthermore, glycosylation pattern on target molecules may be specifically detected by suitable detection molecules. In additional or alternative embodiments further modifications of molecules such as phosphorylation on specific amino acids or functional groups, acetylation on specific groups etc. may be detected.

In further preferred embodiments of the present invention a detection molecule as described herein above, may be conjugated to an entity capable of generating a luminescent, a fluorescent or an electrochemical signal.

Thus, in specific embodiments of the present invention a detection molecule, preferably an antibody, or fragment or derivative thereof may be conjugated or labeled with various markers allowing detection, visualization and/or quantification. This can be accomplished using any suitable labels, which can be conjugated to a detection molecule, using any suitable technique or methods known to the person skilled in the art. Examples of suitable labels include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bioluminescent proteins (e.g. luciferin, luciferase, green fluorsent protein, yellowfluorescent protein, derivatives thereof). A detection molecule may further be labeled with a fluorescent label like 6-FAM, HEX, TET, ROX, Cy3, Cy5, Texas Red and/or at the same time with a quenching label like TAMRA, Dabcyl, Black Hole Quencher, BHQ-1 or BHQ-2. A variety of other useful fluorescents and chromophores are described in Stryer, 1968, Science, 162:526-533. Detection molecules may also preferably be labeled with enzymes or enzymatic domains generating a luminescent, a fluorescent or an electrochemical signal. Particularly preferred examples of such enzymes include horseradish peroxidase, alkaline phosphatase and beta-lactamase. In further embodiments the detection molecules may be labeled with radioisotopes (e.g. ³H, ¹⁴C, ³²P, ³³P, ³⁵S, ¹²⁵I, ¹¹C, ¹³N, ¹⁵O, ¹⁸F, ⁶⁴Cu, ⁶²Cu, ¹²⁴I, ⁷⁶Br, ⁸²Rb, ⁶⁸Ga or ¹⁸F) or particles (e.g. gold). The different types of labels may be conjugated to a detection molecule using various chemistries, e.g. the amine reaction or the thiol reaction. However, other reactive groups than amines and thiols can also be used, e.g. aldehydes, carboxylic acids and glutamine.

In a further specific embodiment the present invention envisages a method of detecting a specific target molecule comprising the provision of a structure or particle comprising a coating as defined herein above, contacting a sample with a structure or particle comprising a coating as defined herein above, and determining the presence of said target molecule in said sample by visualizing or recognizing the binding between the target molecule and said affinity molecule.

In another preferred embodiment of the present invention the detection may be carried out via the detection of clusters comprising at least two magnetic nanoparticles and at least one target molecule. For example, a magnetic nanoparticle as defined herein above may be provided with a coating such that two or more nanoparticles may cluster upon the presence of least one target molecule to be detected, which is capable of bridging or combining both particles. The detection may, in a particularly preferred embodiment be carried out in an optomagnetic system, wherein said magnetic nanoparticles are magnetically actuated and optically detected in a stationary sample fluid, comprising the steps of (i) target molecule capture, e.g. as defined herein above, (ii) magnetic actuation, and (iii) detection.

A "magnetic actuation" as used in the context of the present invention is understood as the application of a uniform magnetic field to a sample containing nanoparticles that have been incubated with a target molecule to detect. Upon the activation of the field the nanoparticles arrange themselves into chains and are free to vibrate and rotate while in close proximity with each other.

The detection of nanoparticles via an optomagnetic system may in particular be performed by the following steps: (1) irradiation of the sample volume with input light;(2) actuation of magnetic nanoparticles as defined above in the sample volume by a magnetic actuation field ; and (3) detection of output light that originates from an interaction of the input light with clusters of said magnetic nanoparticles, in the sample volume. In particular embodiments the said actuation field is at least once interrupted by a pause. This dynamic actuation in terms of repeated pulses has been found to reduce non-specific interactions and enhance the number of binding events. Further details and parameters would be known to the person skilled in the art or can be derived from Ranzoni et al., 2011, Nano Lett., 11, 2017-2022.

In a further alternative embodiment, a detection may be performed via the binding of a coated entity according to the present invention, e.g. a coated particle, preferably a coated nanoparticle, more preferably a coated magnetic nanoparticle, to a sensor surface, e.g. a biosensor. A detection may accordingly be performed by any suitable methodology in the area of biosensors, e.g. methods derivable from Bruls et al., 2009, Lab Chip, , 9, 3504-3510, Yager et al., 2006, Nature, 442 (7101), 412-418, Stern et al,, 2010, Nat. Nanotechnol. 5 (2), 138-142; or Fan et al., 2008, Nat. Biotechnol, 26 (12), 1373-1378. Examples include rotation and scattering methods as described herein, or as known to the person skilled in the art, or derivable from qualified textbooks or documents such as Wild, D., 2005, The immunoassay handbook; Elsevier Science Ltd: New York. Particularly preferred is the employment of methods of optical scattering and/or optical absorption of nanoparticles at a sensing surface. Such methods would be known to the person skilled in the art, or can be derived from Bruls et al., Lab Chip, 2009, 9, 3504-3510.

In another aspect the present invention refers to the use of a coating as defined herein above, e.g. of a single layer, or multiple-layer coating, or of shell structure as defined herein above, the use of a particle as defined herein above, the use of a flat structure as defined herein above, the use of a matrix as defined herein above, or the use of a nanoparticle as defined herein above for the purification of a specific target molecule, e.g. biomolecule, from a sample.

The purification may preferably be performed by providing a structure or particle comprising a coating as defined herein above with a sample as described above comprising one or more target molecules. The target molecule may accordingly be bound by or captured by an affinity molecule as defined herein. Further steps may be employed in order to purify the bound target molecule. For example, the coated structure of particle may be washed, e.g. with suitable washing buffers as known to the person skilled in the art. Such washing steps may be performed one time or repeatedly. Detection molecules as defined herein above may be used, in specific embodiments, in order to determine the amount of bound target molecules and/or the purification grade, e.g. the determination of competitor molecules etc. Subsequently, e.g. after the washing procedure has been terminated, a target molecule bound to an affinity molecule may be eluted from a coated particle or structure, e.g. via appropriate elution buffers, changes in pH or salt concentrations, the employment of heat or of light.

In a further specific embodiment the present disclosure envisages a method of purifying a specific target molecule comprising the provision of a structure or particle comprising a coating as defined herein above, a washing step including washing with suitable washing buffers as known to the person skilled in the art, and a step of eluating a bound target molecule as described above.

In a particularly preferred embodiment the use of the coated entities for purification, or the purification method of the target molecule, e.g. biomolecule as described above may comprise the steps of a (i) capturing a target biomolecule; (ii) washing the target molecule / affinity molecule complex with an appropriate washing buffer, and ; (iii) eluting of the target biomolecule from said matrix, surface or particle as defined herein above.

In specific embodiments of the present disclosure an elution may be carried out with a complex of the target molecule together with the affinity molecule. For example, by using a mild enzymatic elution process a subsequent detection of the protein biomarkers by an affinity assay may become possible. Preferably, such an approach relies on the following steps (see also Figure 6): specific capture by magnetic particles, then concentration into a small volume, and subsequently purification and elution. Finally, the biomarker may be recaptured in an immunoassay for detection. In the first step, antibody coated particles (P), e.g. magnetic particles, in a volume V_{P} are added to the sample volume (V_{S}) containing the biomarker molecules (B). In the combined volume V_{capt}, the biomarker may be captured by the antibodies Ab₁ which may be immobilized on the particles via a DNA linker. The particles may subsequently be transferred to a clean solution with a smaller volume (Vₑ₁), where the biomarker antibody complexes (Ab₁-B) may be eluted from the particles, e.g. by restriction or degradation of the DNA linker. Subsequently, the eluted complex may optionally be detected, for example in a sandwich immunoassay employing two antibodies Ab₂ and Ab₃, binding to different epitopes than Ab₁.

In yet another aspect the present invention refers to the use of a coating as defined herein above, e.g. of a single layer, or multiple-layer coating, or of shell structure as defined herein above, the use of a particle as defined herein above, the use of a flat structure as defined herein above, the use of a matrix as defined herein above, or the use of a nanoparticle as defined herein above for the determination of the concentration of a specific target molecule, e.g. biomolecule, in a sample. The determination of the concentration of a specific target molecule may, for example, be carried out by applying a detection approach as defined herein above in a quantitative manner, e.g. by determining or providing the amount of available affinity molecules, and in particular by carrying out suitable control measurements e.g. based on empty samples or known concentration of a target molecule, or staggered concentrations of a target molecule. In further embodiments of the present invention, the determination may be performed on the basis of purified target molecules obtainable as described herein above. Accordingly, eluted target molecules may be quantified according to any suitable determination method known to the person skilled in the art.

In a further specific embodiment the present invention envisages a method of determining the concentration or amount of a specific target molecule comprising the provision of a structure or particle comprising a coating as defined herein above, a washing step including washing with suitable washing buffers as known to the person skilled in the art, and a step of eluting a bound target molecule as described above, followed by a step of specifically binding the eluted target molecule to a specific affinity molecule. Alternatively, a method may comprise the provision of a structure or particle comprising a coating as defined herein above, a step of contacting a sample with a structure or particle comprising a coating as defined herein above, and determining the presence of said target molecule in said sample by visualizing or recognizing the binding between the target molecule and said affinity molecule, followed or flanked by steps of performing suitable control measurements, e.g. based on empty samples or known concentration of a target molecule, or staggered concentrations of a target molecule as described herein.

In yet another aspect the present invention relates to an assay for the detection of specific target molecules, e.g. biomolecules from a sample and/or determination of the concentration of specific target biomolecules comprising the use of a coating as defined herein above, or the use of a particle, flat structure or matrix comprising a coating as defined herein above, or the use of a nanoparticle as defined herein above.

In a particularly preferred embodiment, said detection of said target biomolecules may be performed in an optomagnetic system, wherein said magnetic nanoparticles are magnetically actuated and optically detected in a stationary sample fluid, comprising the steps of (i) target biomolecule capture, e.g. as defined herein above, (ii) magnetic actuation, and (iii) detection.

For example, the detection of nanoparticles via an optomagnetic system may be performed by the following steps: (1) irradiation of the sample volume with input light;(2) actuation of magnetic nanoparticles as defined above in the sample volume by a magnetic actuation field; and (3) detection of output light that originates from an interaction of the input light with clusters of said magnetic nanoparticles, in the sample volume.

In particular embodiments the said actuation field is at least once interrupted by a pause. This dynamic actuation in terms of repeated pulses has been found to reduce non-specific interactions and enhance the number of binding events. It is further envisaged to modify the pauses, e.g. the duration of a pause and/or the number of pauses. The corresponding values for duration of a pause and/or the number of pauses may additionally be determined from calibration measurements. Preferably, the pause may range between about 0.1 s and about 10 s, more preferably between about 1 s and about 2 s. In a further specific embodiment, the magnetic actuation field may be interrupted by about 10 to about 50 pauses. In yet another preferred embodiment of the present invention the magnetic actuation field may rotate.

In further specific embodiments of the present invention output light may comprise input light which was scattered by clusters of magnetic particles. It is further preferred that the detected clusters are formed by two magnetic particles.

In yet another preferred embodiment said magnetic particles, preferably magnetic nanoparticles may be of two kinds, in particular having different binding sites for a target molecule. Such different binding sites may be provided by the presence of different affinity molecules such as different antibodies binding different epitopes of a target molecule. It is further particularly preferred that said magnetic particles, e.g. magnetic nanoparticles can bind to a target molecule in the sample in a sandwich configuration.

In further embodiments of the present invention, methods or assays as defined herein above, e.g. based on particles, flat structures or matrices comprising a coating, layer structure or shell structure as defined herein above may be used for molecular diagnostics, biological sample analysis, chemical sample analysis, food analysis, and/or forensic analysis.

The following examples and figures are provided for illustrative purposes. It is thus understood that the example and figures are not to be construed as limiting. The skilled person in the art will clearly be able to envisage further modifications of the principles laid out herein.

### EXAMPLES

### Example 1 - Preparation of b-PEG-PSA nanoparticules with modified surface structures

In a typical preparation of nanoparticles having a two-layer shell structure Streptavidin-coated superparamagnetic particles (beads) are used as a starting material, which are commercially available (e.g. Adamtech). In a first step, the magnetic beads were sonicated three times for 3 seconds at 50W. 24 µl of the streptavidin-coated nanoparticle suspension at 10 mg/mL were washed three times in 10 mM PBS buffer and diluted to a final volume of 120 µl (2 mg/ml). The suspension was again sonicated three times for 3 seconds at 50W and subsequently 200 µl PBS buffer was added. In the next step 50 µl of the prepared suspension of streptavidin-coated nanoparticles were incubated for 15 minutes with biotinylated PEG-linker molecules (having a molecular weight of 10 kDa) coupled to PSA (prostate specific antigen) antibody (b-PEG_{10kDa} -PSA). Subsequently, the obtained suspension is additionally incubated for 15 min with a 50 µM solution of non-affine biotinylated spacer molecules (b-PEG_{5kDa}) having a molecular weight of 5 kDa. The obtained suspension was incubated with 11.62 µl Streptavidin (1 mg/ml) for 10 minutes. In the next step, 20µl of 5 mM non-affine biotinylated spacer molecules (b-PEG_{10kDa}-linker) having a molecular weight of 10 kDa was added and incubated for 15 minutes. Optionally, 18.6 µl of 5 mM b-PEG_{5kDa}-spacer molecules are used. The resulting nanoparticle solution was washed three times in assay buffer and subsequently added with buffer to a final volume of 100 µl. The final suspension was sonicated three times for 3 seconds at 50W.

### Example 2 - Zeta potential and mean diameter of nanoparticles with different surface modifications

In this example, properties of the nanoparticles with different surface modifications have been assessed. The nanoparticles have been prepared as described in the protocol of Example 1. The non-affine spacer molecule were purchased from CreativePegWorks at a range higher than 3.5 kDa, which corresponds to a length of approximately 40 nm. The linker molecules were also purchased from CreativePegWorks. These molecules are at one end coupled to biotin and at the other end to a N-hydroxysuccinimide (NHS)-group. Biotinylated polyethylene glycol (PEG) linkers have been conjugated to antibodies against PSA (Prostate Specific Antigen) by means of NHS-chemistry. The bioconjugation has been performed such that more than one PEG- linker is coupled to the antibody. The biotinylated antibodies (25 µg/mg) are incubated with streptavidin-coated nanoparticles together with non-affine spacer molecules. The amount of antibodies per particle can be tuned by the ratio of the solution concentration of antibody-containing linker molecules and non-affine spacer molecules. The antibody concentration was selected such that a maximum coverage as described by the nanoparticle manufacturer (Masterbeads 500nm, StreptaDivin coated, Ademtech - binding capacity of avidin according to specifications > 1nM). The concentration of non-affine spacer molecules was selected such that a maximum loading of biotin per the streptavidin coated particle was obtained. Full coverage of the nanoparticle surface with biotin is necessary in order to avoid cross-linking of particles. The dense three-dimensional structure of the second layer was formed by the multivalent binding properties of streptavidin, which due to its tetrameric structure is capable of binding four biotin molecules. The multivalency of streptavidin thus leads to a mesh of non-affine spacer molecules as depicted in the right panel of Figure 1.

The properties of nanoparticles with different surface modification were then analyzed by dynamic light scattering (DLS). In particular, the charge at the surface of the produced nanoparticles and the average hydrodynamic radius was measured. The results are depicted in Figure 2. The diagram in the left panel shows that the negative charge on the nanoparticles decreases progressively with increasing length (molecular weight) of the linker/spacer molecules loaded onto the nanoparticle, i.e. increasing thickness of the surface coating. The charge (Z-potential) reaches a minimum value for the double-layer configuration. This also indicates a lower colloidal stability since the electrostatic charge is considerably lowered by the presence of linker-molecules. Figure 2B demonstrates that at the same time the mean diameter considerably increases with increasing length (molecular weight) of the linker/spacer molecules loaded onto the nanoparticle. While the bare nanoparticles have a diameter of around 510 nm, the nanoparticles with a double-layer coating have a diameter of around 570 nm.

### Example 3 - Determination of non-specific and specific particle-particle binding using a cluster-based optomagnetic assay

In order to assess clustering of magnetic particles a bionanotechnique was used as described in Ranzoni et al., 2011, Nano Lett, 11, 2017-2022. This optomagnetic technique was applied to detect biomolecules in fluid by interparticle binding. The assay allows determination of signals deriving from non-specific and specific particle-particle binding.

When antibodies are directly coupled to the surface of magnetic nanoparticles, i.e. without a linker, the amount of is substantially high. Hence, directly coupled nanoparticles can only be used in such an assay, when surfactants are present in the assay buffer to diminish non-specific interactions. Figure 3A shows a dose-response curve measured with this bionanotechnique using nanoparticles as are commonly available, i.e. where the antibodies are directly bound without any linker and coating. The thicker horizontal line indicates the blank signal, which constitutes the limiting factor when determining the limit of detection. The detection limit is determined by non-specific binding processes of the nanoparticles. For the uncoated nanoparticles the blank level was approximately 160 V/Hz^{0.5}.

In contrast, if nanoparticles with a double-layer architecture as prepared in Examples 1 or 2 are used in this assay, the blank signal is significantly lowered. As can be seen in Figure 3B, the background level is reduced to less than 80 V/Hz^{0.5}, i.e. by a factor of 2. Hence, while in the configuration without a surface coating the limit of detection was determined to be approximately at 5 pM (see Figure 3A), the double-layer architecture was able to considerably improve this value to approximately 200 fM (see Figure 3B). Importantly, the measurement of nanoparticles with modified surface structures does therefore not require the presence of a surfactant. Such a reduction in blank level thus represents a remarkable improvement in terms of dynamic range and limit of detection.

### Example 4 - Determination of non-specific and specific particle-particle binding in a complex biological matrix

This Example also used the optomagnetic setup as described in Ranzoni et al., 2011, Nano Lett, 11, 2017-2022. Analytical assays are particularly challenging in biological matrices such as human plasma. In comparison to assay buffer, human plasma contains large quantities of potentially interfering agents and biomolecules, which can non-specifically bind to nanoparticles and cause severe clustering. As has been mentioned above, the non-specific clustering leads to a higher blank signal and negatively affects the limit of detection in this assay.

In this Example, 95% human plasma pool was used, which was previously depleted from Prostate Specificity Antigen (PSA) by a pull-down with anti-PSA-coated beads. The diagram in Figure 4 shows the dose-response curve of anti-PSA-coated nanoparticles having a double-layer shell structure as described in Examples 1 and 2. Even without the use of a surfactant, the measured blank signal in such a complex matrix is at 140 V/Hz^{0.5}. Remarkably, the measured background signal was thus even lower than the blank value of uncoated nanoparticles in a surfactant-containing assay buffer (see Example 3 and Figure 3A). The saturation of the signal at higher concentrations can be explained by the fact that plasma is approximately 5 times more viscous than buffer, which makes magnetic actuation difficult. However, the slope and the performance characteristics at low concentrations of PSA are almost identical to the comparative measurement in buffer. The limit of detection was at still less than 0.7 pM.

### Example 5 - Determination of non-specific and specific particle-particle binding in untreated plasma pool

The same experimental setup as in Example 4 was applied, however with the difference that untreated plasma pool from female donors has been investigated. The plasma contained 2% w/v Pluronic F127. In this example, a depletion of PSA as described in Example 4 is unnecessary since plasma from female donors lack PSA. The ratio behind this experiment is that by the depletion of PSA in Example 4 also a certain amount of non-specific interacting molecules is removed from the plasma. Hence, this experimental setup provides a situation where no pretreatment has been performed.

As can be seen in Figure 5, the improved surface architecture of the nanoparticles leads to a limit of detection of 0.7 pM, although the amount of non-specific interacting molecules in untreated plasma is much higher. Hence, blank level was not negatively affected in this experimental setup, suggesting that the double-layer coating effectively prevents non-specific interactions and enhances colloidal stability of the nanoparticles also in non-depleted complex matrices. The dose-response curve in Figure 5A shows two slopes. The signal level at low concentrations corresponds to species of two-particle actuators, whereas the slope at concentrations higher than 10 pM corresponds to clusters of more than two particles. Hence, the pivot point at 10 pM represents a transition from two particle cluster to a more widespread size distribution with larger cluster sizes. Figure 5 also shows the frequency response at concentration of 1 pM (Figure 5B) and 25 pM (Figure 5C) of PSA. The curve at 1 pM shows one critical transition at approximately 4 Hz whereas the measurement at 25pM shows a shift of the critical transition to approximately 1Hz. The shift is due to the formation of larger clusters in the solution and it is triggered by the higher concentration of PSA. Such a level of control in this assays system demonstrates the capability of the nanoparticle coating as described herein to prevent non-specific particle clustering in complex matrices.

## Claims

1. A coating comprising a shell structure, wherein said shell structure comprises:
- a first layer comprising one or more affinity molecules for capturing a specific target biomolecule in a sample, wherein the one or more affinity molecules are conjugated to a non-affine spacer molecule, wherein the non-affine spacer molecules comprise a molecule selected from the group consisting of peptides, nucleic acids, dendrimers, dendrons, polyethylene glycol, or a mixture thereof,
- wherein the bioconjugation has been performed such that more than one non-affine spacer molecule is coupled to the affinity molecule,
- a second layer, which is coupled to the first layer, comprising non-affine spacer molecules and
- multivalent binding molecules with multiple binding sites, such as streptavidin,
Wherein first and second layer further comprise
an interaction partner, such as biotin, bound to said non affine-spacer molecules, for interacting with the multivalent binding molecules, wherein a mesh of non-affine spacer molecules is formed by the interaction of the multivalent binding molecules and interaction partners,
wherein said one or more affinity molecules are embedded within the coating structure and wherein said mesh generates a steric hindrance for non-specific molecules, and wherein the first layer is to be coupled to a particle surface, a flat surface, matrix, or a nanoparticle surface

2. The coating of claim 1, wherein said affinity molecule is selected from the group consisting of aptamers, peptides, proteins, oligonucleotides, and molecular imprinted polymers, wherein said affinity molecule preferably is an antibody or a fragment thereof.

3. The coating of claim 1 or 2, wherein the material of said surfaces or said matrix preferably comprises a material selected from the group consisting of agarose, polystyrene, latex, polyvinyl alcohol, silica and a magnetic material.

4. The coating of any one of claims 1 to 3, wherein said multivalent binding molecule with multiple binding sites is a protein.

5. The coating of any one of claims 1 to 4, wherein said multivalent binding molecule with multiple binding sites is streptavidin, and wherein the corresponding interaction partner is biotin.

6. A particle, preferably a nanoparticle, a flat structure or a matrix comprising a coating according to any one of claims 1, 2, 4 or 5, preferably comprising a material selected from the group consisting of agarose, polystyrene, latex, polyvinyl alcohol, silica and a magnetic material, wherein the first layer is coupled to the particle, flat structure, matrix, or nanoparticle surface.

7. The particle of claim 8, which is a magnetic nanoparticle.

8. Use of the coating of any of one of claims 1 to 5, the particle, flat structure or matrix of claim 6, or the nanoparticle of claim 7 for detection and optionally purification of a specific target biomolecule from a sample; and/or determination of the concentration of the specific target biomolecule.

9. The use of claim 8, wherein said specific target biomolecule is captured by said affinity molecule, and wherein said specific target biomolecule is further recognized by a detection molecule, which is preferably conjugated to an enzyme capable of generating a luminescent, a fluorescent or an electrochemical signal, wherein said detection molecule is preferably an antibody or a fragment thereof.

10. The use of claim 8 or 9, wherein the specific target biomolecule is detected via detection of clusters comprising at least two magnetic nanoparticles and at least one target biomolecule, or detected via binding to a detection molecule on a sensor surface.

11. Use of the coating of any one of claims 1, 2, 4 or 5 for
i) improving the specificity of a preexisting surface layer comprising affinity molecules, and/or
ii) preventing or minimizing non-specific binding to said preexisting surface layer,
wherein said coating is attached on top of a preexisting layer of affinity molecules.

12. An assay for the detection of specific target biomolecules from a sample and/or determination of the concentration of specific target biomolecules comprising the use of the coating of any of claims 1 to 5, the particle, flat structure or matrix of claim 6 , or the nanoparticle of claim 7.

13. The assay of claim 12, wherein detection of said target biomolecules is performed in an optomagnetic system, wherein said magnetic nanoparticles are magnetically actuated and optically detected in a stationary sample fluid, comprising the steps of
i) target biomolecule capture,
ii) magnetic actuation, and
iii) detection.

## Patentansprüche

1. Beschichtung mit einer Mantelstruktur, wobei die Mantelstruktur umfasst:
- eine erste Schicht mit einem oder mehreren Affinitätsmolekülen zum Erfassen eines spezifischen Ziel-Biomoleküls in einer Probe, wobei das eine oder mehrere Affinitätsmoleküle mit einem nicht-affinen Spacer-Molekül konjugiert sind, wobei die nicht-affinen Spacer-Moleküle ein Molekül umfassen, das aus der Gruppe, bestehend aus Peptiden, Nukleinsäuren, Dendrimeren, Dendrons, Polyethylenglycol oder einer Mischung daraus, ausgewählt wird,
wobei die Biokonjugation so durchgeführt wurde, dass mehr als ein nichtaffines Spacer-Molekül an das Affinitätsmolekül gekoppelt wird,
- eine zweite Schicht, die mit der ersten Schicht gekoppelt ist, mit nicht-affinen Spacer-Molekülen und
- multivalenten Bindungsmolekülen mit mehreren Bindungsstellen, wie zum Beispiel Streptavidin,
wobei die erste und zweite Schicht umfassen:
einen Interaktionspartner, wie zum Beispiel an die nicht-affinen Spacer-Moleküle gebundenes Biotin, um mit den multivalenten Bindungsmolekülen zu interagieren,
wobei durch die Interaktion der multivalenten Bindungsmoleküle und Interaktionspartner ein Netz aus nicht-affinen Spacer-Molekülen gebildet wird,
wobei das eine oder mehrere Affinitätsmoleküle innerhalb der Beschichtungsstruktur eingebettet sind, und wobei das Netz eine sterische Hinderung für nicht-spezifische Moleküle erzeugt, und wobei die erste Schicht mit einer Partikeloberfläche, einer flachen Oberfläche, einer Matrix oder einer Nanopartikeloberfläche zu koppeln ist.

2. Beschichtung nach Anspruch 1, wobei das Affinitätsmolekül aus der Gruppe, bestehend aus Aptameren, Peptiden, Proteinen, Oligonucleotiden sowie molekular geprägten Polymeren, ausgewählt wird, wobei das Affinitätsmolekül vorzugsweise ein Antikörper oder ein Fragment desselben ist.

3. Beschichtung nach Anspruch 1 oder 2, wobei das Material der Oberflächen oder der Matrix vorzugsweise ein Material enthält, das aus der Gruppe, bestehend aus Agarose, Polystyrol, Latex, Polyvinylalkohol, Siliziumdioxid sowie einem magnetischen Material, ausgewählt wird.

4. Beschichtung nach einem der Ansprüche 1 bis 3, wobei das multivalente Bindungsmolekül mit mehreren Bindungsstellen ein Protein ist.

5. Beschichtung nach einem der Ansprüche 1 bis 4, wobei das multivalente Bindungsmolekül mit mehreren Bindungsstellen Streptavidin ist, und wobei der entsprechende Interaktionspartner Biotin ist.

6. Partikel, vorzugsweise ein Nanopartikel, eine flache Struktur oder eine Matrix mit einer Beschichtung nach einem der Ansprüche 1, 2, 4 oder 5, das vorzugsweise ein Material umfasst, das aus der Gruppe, bestehend aus Agarose, Polystyrol, Latex, Polyvinylalkohol, Siliziumdioxid sowie einem magnetischen Material, ausgewählt wird, wobei die erste Schicht mit dem Partikel, der flachen Struktur, der Matrix oder der Nanopartikeloberfläche gekoppelt ist.

7. Partikel nach Anspruch 6, bei dem es sich um ein magnetisches Nanopartikel handelt.

8. Verwendung der Beschichtung nach einem der Ansprüche 1 bis 5, des Partikels, der flachen Struktur oder Matrix nach Anspruch 6 oder des Nanopartikels nach Anspruch 7 zur Detektion und, optional, Reinigung eines spezifischen Ziel-Biomoleküls aus einer Probe; und/oder Ermitteln der Konzentration des spezifischen Ziel-Biomoleküls.

9. Verwendung nach Anspruch 8, wobei das spezifische Ziel-Biomolekül durch das Affinitätsmolekül erfasst wird, und wobei das spezifische Ziel-Biomolekül weiterhin von einem Detektionsmolekül identifiziert wird, das vorzugsweise mit einem Enzym konjugiert ist, das imstande ist, ein lumineszierendes, ein fluoreszierendes oder ein elektrochemisches Signal zu erzeugen, wobei das Detektionsmolekül vorzugsweise ein Antikörper oder ein Fragment desselben ist.

10. Verwendung nach Anspruch 8 oder 9, wobei das spezifische Ziel-Biomolekül durch Detektion von Clustern mit mindestens zwei magnetischen Nanopartikeln und mindestens einem Ziel-Biomolekül detektiert wird oder durch Bindung an ein Detektionsmolekül auf einer Sensoroberfläche detektiert wird.

11. Verwendung der Beschichtung nach einem der Ansprüche 1, 2, 4 oder 5, um
i) die Spezifität einer präexistenten Oberflächenschicht mit Affinitätsmolekülen zu verbessern, und/oder
ii) nicht-spezifische Bindung an die präexistente Oberflächenschicht zu verhindern oder zu minimieren,
wobei die Beschichtung auf der Oberseite einer präexistenten Schicht von Affinitätsmolekülen angelagert ist.

12. Assay zur Detektion spezifischer Ziel-Biomoleküle aus einer Probe und/oder Ermitteln der Konzentration spezifischer Ziel-Biomoleküle, umfassend die Verwendung der Beschichtung nach einem der Ansprüche 1 bis 5, des Partikels, der flachen Struktur oder Matrix nach Anspruch 6 oder des Nanopartikels nach Anspruch 7.

13. Assay nach Anspruch 12, wobei die Detektion der Ziel-Biomoleküle in einem optomagnetischen System durchgeführt wird, wobei die magnetischen Nanopartikel magnetisch aktiviert und in einer stationären Probenflüssigkeit optisch detektiert werden, was die folgenden Schritte beinhaltet:
i) Ziel-Biomolekül-Erfassung,
ii) magnetische Aktivierung, sowie
iii) Detektion.

## Revendications

1. Revêtement comprenant une structure d'enveloppe, dans lequel ladite structure d'enveloppe comprend :
- une première couche comprenant une ou plusieurs molécules d'affinité pour capturer une biomolécule cible spécifique dans un échantillon, dans lequel la ou les molécules d'affinité sont conjuguées à une molécule d'espacement non affine, dans lequel les molécules d'espacement non affines comprennent une molécule choisie dans le groupe constitué de peptides, acides nucléiques, dendrimères, dendrons, polyéthylène glycol, ou un mélange de ceux-ci,
- dans lequel la bioconjugaison a été exécutée de telle sorte que plus d'une molécule d'espacement non affine est couplée à la molécule d'affinité,
- une seconde couche, qui est couplée à la première couche, comprenant des molécules d'espacement non affines et
- des molécules de liaison multivalentes avec plusieurs sites de liaison, telles que la streptavidine,
dans lequel les première et seconde couches comprennent en outre
un partenaire d'interaction, tel que de la biotine, lié auxdites molécules d'espacement non affines, pour interagir avec les molécules de liaison multivalentes, dans lequel un maillage de molécules d'espacement non affines est formé par l'interaction des molécules de liaison multivalentes et des partenaires d'interaction,
dans lequel ladite ou lesdites molécules d'affinité sont intégrées dans la structure de revêtement et dans lequel ledit maillage produit un encombrement stérique pour des molécules non spécifiques et dans lequel la première couche est destinée à être couplée à une surface de particule, une surface plate, une matrice, ou une surface de nanoparticule.

2. Revêtement selon la revendication 1, dans lequel ladite molécule d'affinité est choisie dans le groupe constitué d'aptamères, peptides, protéines, oligonucléotides et polymères à empreintes moléculaires, dans lequel ladite molécule d'affinité est de préférence un anticorps ou un fragment de celui-ci.

3. Revêtement selon la revendication 1 ou 2, dans lequel le matériau desdites surfaces ou de ladite matrice comprend de préférence un matériau choisi dans le groupe constitué d'agarose, polystyrène, latex, alcool polyvinylique, silice et un matériau magnétique.

4. Revêtement selon l'une quelconque des revendications 1 à 3, dans lequel ladite molécule de liaison multivalente avec plusieurs sites de liaison est une protéine.

5. Revêtement selon l'une quelconque des revendications 1 à 4, dans lequel ladite molécule de liaison multivalente avec plusieurs sites de liaison est la streptavidine, et dans lequel le partenaire d'interaction correspondant est la biotine.

6. Particule, de préférence une nanoparticule, structure plate ou matrice comprenant un revêtement selon l'une quelconque des revendications 1, 2, 4 ou 5, comprenant de préférence un matériau choisi dans le groupe constitué d'agarose, polystyrène, latex, alcool polyvinylique, silice et un matériau magnétique, dans laquelle la première couche est couplée à la surface de la particule, de la structure plate, de la matrice ou de la nanoparticule.

7. Particule selon la revendication 8, qui est une nanoparticule magnétique.

8. Utilisation du revêtement selon l'une quelconque des revendications 1 à 5, de la particule, structure plate ou matrice selon la revendication 6, ou de la nanoparticule selon la revendication 7, pour la détection et éventuellement la purification d'une biomolécule cible spécifique d'un échantillon ; et/ou la détermination de la concentration de la biomolécule cible spécifique.

9. Utilisation selon la revendication 8, dans laquelle ladite biomolécule cible spécifique est capturée par ladite molécule d'affinité, et dans laquelle ladite biomolécule cible spécifique est en outre reconnue par une molécule de détection, qui est de préférence conjuguée à une enzyme capable de générer un signal luminescent, fluorescent ou électrochimique, dans laquelle ladite molécule de détection est de préférence un anticorps ou un fragment de celui-ci.

10. Utilisation selon la revendication 8 ou 9, dans laquelle la biomolécule cible spécifique est détectée par l'intermédiaire d'une détection de groupements comprenant au moins deux nanoparticules magnétiques et au moins une biomolécule cible, ou est détectée par l'intermédiaire d'une liaison à une molécule de détection sur une surface de capteur.

11. Utilisation du revêtement selon l'une quelconque des revendications 1, 2, 4 ou 5 pour
i) améliorer la spécificité d'une couche de surface préexistante comprenant des molécules d'affinité, et/ou
ii) empêcher ou réduire au minimum la liaison non spécifique à ladite couche de surface préexistante, dans laquelle ledit revêtement est fixé au-dessus d'une couche préexistante de molécules d'affinité.

12. Dosage pour la détection de biomolécules cibles spécifiques d'un échantillon et/ou la détermination de la concentration des biomolécules cibles spécifiques, comprenant l'utilisation du revêtement selon l'une quelconque des revendications 1 à 5, de la particule, structure plate ou matrice selon la revendication 6 ou de la nanoparticule selon la revendication 7.

13. Dosage selon la revendication 12, dans lequel la détection desdites biomolécules cibles est effectuée dans un système optomagnétique, dans lequel lesdites nanoparticules magnétiques sont activées magnétiquement et détectées optiquement dans un fluide échantillon fixe, comprenant les étapes de
i) capture de biomolécule cible,
ii) activation magnétique, et
iii) détection.
